# EUROPEAN PATENT APPLICATION

(11) **EP 1 362 585 A2**
(43) Date of publication of application: **19.11.2003**
(21) Application number: 02711756.3
(22) Date of filing: 25.01.2002
(51) Int. Cl.: A61K 31/00, A61P 29/00, A61P 21/02

(54) **PHARMACEUTICAL COMPOSITIONS CONTAINING A COX-II INHIBITOR AND A MUSCLE RELAXANT**

(30) Priority: 26.01.2001 US 770901
(71) Applicant: Osmotica Corp., Tortola (VG)
(72) Inventor: FAOUR, Joaquina, 1426 Buenos Aires (AR); VERGEZ, Juan A., 1643 Buenos Aires (AR)
(74) Representative: Weber, Thomas, Dr.
(86) International application number: CR0200001
(87) International publication number: WO02058620

(57) **Abstract**

The present invention provides a pharmaceutical composition and dosage form containing in combination a COX-II inhibitor and a muscle relaxant. The pharmaceutical composition is useful for the treatment of pain and pain related disorders and symptoms. The combination provides an improved therapeutic response as compared to either drug alone. The pharmaceutical composition can be included in any dosage form.

## Description

### FIELD OF THE INVENTION

This invention pertains to pharmaceutical compositions comprising a cyclooxygenase-II (COX-II) inhibitor and a muscle relaxant. More particularly, it pertains to formulations that provide therapeutically effective levels of a COX-II inhibitor and a muscle relaxant and their use in treating pain, especially muscle pain.

### BACKGROUND OF THE INVENTION

COX-II inhibitors are widely known for the treatment of pain and pain related disorders and diseases. These compounds have been administered either alone or together with analgesics or NMDA inhibitors. Known pharmaceutical compositions containing a COX-II inhibitor include a cream, pill, tablet and other such dosage forms.

Muscle relaxants have been evaluated alone or in combination with conventional analgesics for the treatment of pain. Mixed and unpredictable results have been obtained. Muscle relaxants have not previously been combined with a COX-II inhibitor in a pharmaceutical composition. Pridinol, a muscle relaxant, is a known antiparkinson/anticholinergic agent used in the treatment of Parkinson's disease.

It is well known that drugs used in the same therapeutic area or even for treating the same indication cannot always be combined *a priori* with the expectation of at least additive therapeutic effects. The scientific literature is full of examples wherein compounds of different classes, which are used to treat the same indication, cannot be combined into safe and efficacious dosage forms thereby resulting in incompatible drug combinations. The reasons for this unexpected lack of compatibility are varied; however, it is often found that the incompatible drug combinations result in increased side effects, unwanted drug interactions or new side effects. More specifically, in the area of analgesia, there are analgesic drug combinations that are contraindicated for some or all of these very same reasons.

Conventional analgesic therapy generally involves administration of a pharmaceutical composition containing one or two different analgesic drugs. However, not all combinations of analgesic drugs are more suitable, in terms of safety or efficacy, than the administration of a single product. Furthermore, the additivity of the analgesic effect of analgesic drugs cannot be predicted *a priori.* For example, M. R. Naidu et al. *(Pharmacotherapy* (1994), Mar.-Apr., 14(2), pp. 173-177) report that the administration of ketorolac alone is superior in terms of analgesia to the combined administration of ibuprofen and paracetamol in the same or different dosage forms for the relief of postoperative pain. In addition, R. Dionne *(Compend. Contin. Educ. Dent.* (2000) July, 21(7), pp. 572-574 and 576-577) reports that the combination of an opioid with acetaminophen or aspirin does not provide greater analgesia but results in a higher incidence of side effects such as drowsiness and nausea. Moreover, S. Ilkjaer et al. *(Acta Anaesthesiol. Scand.* (2000), Aug., 44(7), pp. 873-877) report that the combination of ibuprofen with dextromethorphan provides no additive analgesic effect.

The discovery or expectation of a synergistic analgesic effect from a combination of analgesic drugs or drug classes is also unpredictable. G. L. Wideman et al. report that, when hydrocodone is administered with ibuprofen to a subject for the treatment of postoperative pain, an additive and not synergistic analgesic effect is found. R. A. Dionne *(J*. *Oral Maxillofac. Surg.* (1999), June, 57(6), pp. 673-678) reports that the combined administration of an nonsteroidal anti-inflammatory drug (NSAID), such as ibuprofen, and an orally effective opioid analgesic (such as oxycodone) to patients for the treatment of post-operative oral surgery provides an additive and not synergistic analgesic effect. S.M. Siddik et al. (*Reg. Anesth. Pain Med.* (2001), July-Aug., 26(4), pp. 310-315) report the results of a comparative study on the analgesic effects provided by morphine in combination with propacetamol and/or diclofenac. The combination of diclofenac and morphine provides improved analgesia and resulted in reduced morphine demand, whereas the combination of propacetamol and morphine did not improve analgesia or reduce the demand for morphine significantly. In addition, the combination of diclofenac, propacetamol and morphine did not even provide an additive analgesic effect.

To date, no pharmaceutical compositions or dosage forms comprising a combination of a COX-II inhibitor and a muscle relaxant, in particular pridinol, have been made.

### SUMMARY OF THE INVENTION

The present invention provides a new therapeutic method, composition therefor, and dosage forms therefor, for the treatment of muscle pain and other pain related diseases and disorders and for the treatment or prevention of pain symptoms.

In one aspect, the present invention provides a pharmaceutical composition comprising:
a COX-II inhibitor;
a muscle relaxant; and
at least one pharmaceutical excipient.

Specific embodiments of the invention include those wherein: 1) the pharmaceutical composition is contained within a dosage form such as a gel, cream, ointment, pill, tablet, capsule, liquid, suspension, osmotic device, bead, granule, spheroid, particulate, paste, prill, reconstitutable solid, powder, or injectable liquid; 2) the pharmaceutical composition is adapted for oral, buccal, ocular, otic, dermal, rectal, vaginal, parenteral, sublingual, nasal, or pulmonary delivery; 3) the muscle relaxant is selected from the group consisting of alcuronium, alosetron, aminophylline, baclofen, carisoprodol (SOMA®), chlorphenesin, chlorphenesin carbamate, chlorzoxazone (PARAFON FORTE®), chlormezanone, cyclobenzaprine (FLEXERIL®), dantrolene, decamethonium, diazepam, dyphylline, eperisione, ethaverine, gallamine triethiodide, hexafluorenium, mephenesin, metaxalone (SKELAXIN®), methocarbamol (ROBAXIN®), metocurine iodide, orphenadrine (NORFLEX®), pancuronium, papaverine, pipecuronium, pridinol (pridinolum), succinylcholine, theophylline, tizanidine, tolperisone, tubocurarine, vecuronium, idrocilamide, ligustilide, cnidilide, and senkyunolide; 4) the pharmaceutical composition is a solid dosage form that independently provides a controlled, delayed, sustained, immediate, timed, slow or rapid release of each of the COX-II inhibitor and the muscle relaxant; 5) the pharmaceutical composition provides therapeutically effective plasma levels of the COX-II inhibitor and muscle relaxant for a period of at least 12 hours after administration; and/or 6) the COX-II inhibitor is selected from the group consisting of rofecoxib (VIOXX™, MK-0966), celecoxib (CELEBREX™, SC-58635), flosulide (CGP-28238), NS-398, DUP-697, meloxicam, 6-methoxy-2-naphthylacetic acid (6-MNA), nabumetone (prodrug for 6-MNA), etodolac, nimesulide, SC-5766, SC-58215, T-614 and combinations thereof.

Another aspect of the invention provides a controlled release combination device comprising:
a core comprising a therapeutically effective amount of a COX-II inhibitor and at least one osmotic agent or osmopolymer, wherein the core provides a controlled release of the COX-II inhibitor;
a semipermeable membrane surrounding the core and having a passageway there through; and
an external coat comprising a therapeutically effective amount of a muscle relaxant, wherein the external coat provides a rapid release of the muscle relaxant; and wherein:
   at least 75% of the COX-II inhibitor is released within 24 hours, and at least 75 % of the muscle relaxant is released within 40 minutes after exposure of the osmotic device to an aqueous solution.

In other embodiments, the external coat is applied by spray coating rather than by compression coating. By spray coating rather than compression coating the external coat is thinner, and therefore a smaller osmotic device is formed.

Other embodiments include those wherein: 1) the controlled release device further comprises an inert water soluble or erodible lamina interposed the semipermeable membrane and the drug-containing outer coating; 2) the water soluble lamina comprises poly(vinylpyrrolidone)-(vinyl acetate) copolymer; and/or 3) the controlled release device is an osmotic device.

Yet another aspect of the invention provides a rapid release dosage form comprising a COX-II inhibitor and a muscle relaxant, wherein each drug is released rapidly and the dosage form provides therapeutically effective levels of each drug for a period of at least 12 hours, a period of 12 to 60 hours, a period of 12 to 30 hours, or a period of 18 to 48 hours. The plasma levels of drug are either independent of or dependent upon one another.

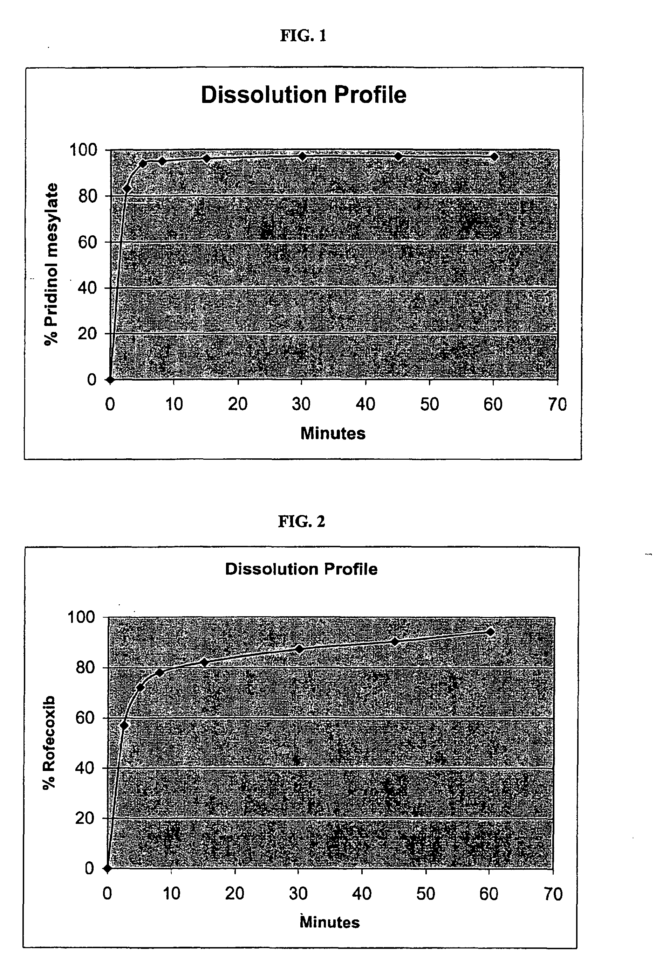

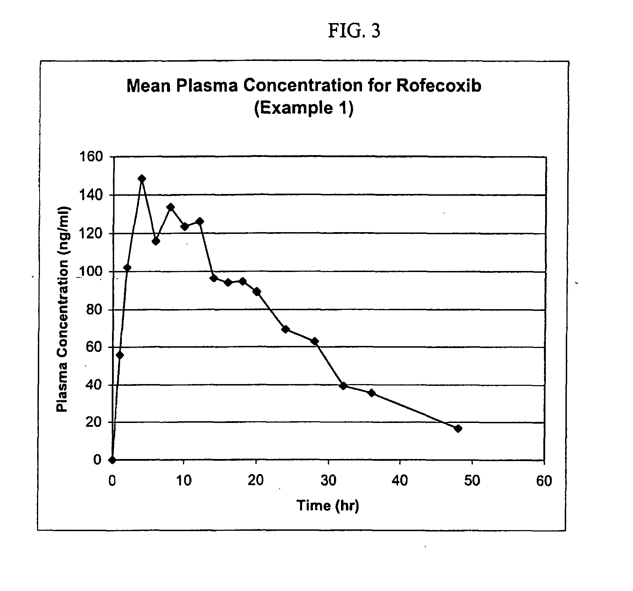

Specific embodiments of the invention include those wherein: 1) the pharmaceutical composition of the invention provides drug release profiles similar to that depicted in FIGS. 1-2; 2) the pharmaceutical composition provides therapeutic plasma levels for the muscle relaxant in an amount sufficient to provide a therapeutic benefit to a subject to whom the pharmaceutical composition is administered; 3) the pharmaceutical composition provides therapeutic plasma levels for the COX-II inhibitor generally in the range of about 90 ng to about 300 ng per ml of plasma; and/or 4) the pharmaceutical composition of the invention provides plasma concentration profiles in a mammal similar to that depicted in FIGS. 3-4.

Another aspect of the invention provides a method of treating a pain related disorder, such as muscle pain, acute and chronic pain, pain from chemoprevention, dental pain, dysmenorrhea, gout, headache, tendonitis, bursitis, rheumatoid arthritis, and osteoarthritis in a mammal. The method comprises the step of administering a pharmaceutical composition comprising a COX-II inhibitor, a muscle relaxant and at least one pharmaceutical excipient, wherein the pharmaceutical composition provides therapeutically effective levels of the drugs when administered to a mammal.

Other features, advantages and embodiments of the invention will become apparent to those skilled in the art by the following description, figures and accompanying examples.

### BRIEF DESCRIPTION OF THE FIGURES

The following drawings are part of the present specification and are included to further demonstrate certain aspects of the invention. The invention may be better understood by reference to one or more of these drawings in combination with the detailed description of the specific embodiments presented herein.
FIG. 1 depicts an exemplary *in vitro* dissolution profile for pridinol mesylate as it is released from an immediate/rapid release tablet made according to Example 1.
FIG. 2 depicts an exemplary *in vitro* dissolution profile of rofecoxib as it is released from the immediate/rapid release tablet of FIG. 1.
FIG. 3 depicts an exemplary *in-vivo* plasma concentration profile for rofecoxib as provided by the formulation of Example 1.
FIG. 4 depicts an exemplary *in-vivo* plasma concentration profile including the minimum and maximum plasma concentrations for rofecoxib as provided by the formulation of Example 1.

### DETAILED DESCRIPTION OF THE INVENTION

The present invention provides a pharmaceutical composition useful for and a method of treating pain related disorders, in particular muscular or skeletal pain disorders, such as muscle pain, arthritis, osteoarthritis, inflammation, muscle pain, acute and chronic pain, chemoprevention, dental pain, dysmenorrhea, gout, headache, tendonitis, bursitis, and rheumatoid arthritis. The pharmaceutical composition provides relief of pain symptoms when administered to a mammal.

The claimed composition and dosage form, which include a combination of a COX-II inhibitor and a muscle relaxant, unexpectedly provide an improved, additive or synergistic analgesic effect when administered to a subject as compared to the analgesic effect provided by the administration of either agent alone.

The pharmaceutical composition of the invention includes a COX-II inhibitor in combination with a muscle relaxant. When the pharmaceutical composition is included in a dosage form, each unit dose will contain therapeutically effective amounts of the COX-II inhibitor and/or the muscle relaxant. Additionally, each unit dose will provide therapeutically effective plasma levels of the COX-II inhibitor and/or the muscle relaxant. Depending upon the particular combination of COX-II inhibitor and muscle relaxant used, an improved (enhanced), additive or synergistic therapeutic effect will be observed.

The dosage form in which the pharmaceutical composition is present will depend upon the specific COX-II inhibitor and muscle relaxant used and the intended mode of administration. The COX-II inhibitor is independently selected at each occurrence from any of the COX-II inhibitors disclosed herein or known in the art. The muscle relaxant is independently selected at each occurrence from any of the muscle relaxants disclosed herein or known in the art. Accordingly, both the COX-II inhibitor and the muscle relaxant are independently selected at each occurrence.

The release profile for each of the COX-II inhibitor and muscle relaxant, as provided by a unit dose containing the same, may be dependent or independent of one another. In other words, in a unit dose containing the COX-II inhibitor and the muscle relaxant, the release profile of the muscle relaxant may be independent or dependent of the release profile of the COX-II inhibitor.

Different environments for use of the pharmaceutical composition include biological environments such as the oral, ocular, nasal, vaginal, glands, gastrointestinal tract, rectum, cervical, intrauterine, arterial, venous, otic, sublingual, dermal, epidermal, subdermal, implant, buccal, bioadhesive, mucosal, and parenteral environments.

In one embodiment, the COX-II inhibitor and the muscle relaxant are released concurrently. This type of release occurs when the two drugs are included together in admixture, for example, in a tablet core, powder, capsule, bead, granule, liquid, paste, gel, cream, ointment, patch, implant and other similar dosage forms capable of simultaneously delivering two or more drugs.

In another embodiment, the COX-II inhibitor and the muscle relaxant are released sequentially. This type of release occurs when the first drug is included in one part of a dosage form and the second drug is included in another part of the same dosage form, and release of the second drug begins shortly after or nearly at the end of completion of release of the first drug. Such dosage form would include those wherein the first drug is included in a core and the second drug is included in a coat surrounding the core, a bilayered tablet with each drug being in a different core, a dosage form providing a rapid release of the first drug and a controlled release of the second drug. Suitable dosage forms for this embodiment include, for example, a layered patch, layered or coated tablet or bead, layered or coated osmotic device, capsule containing a mixture of beads that provide different release profiles for the drugs, and layered or coated implant.

In yet another embodiment, the COX-II inhibitor and the muscle relaxant are released in spaced apart periods of time. This type of release occurs when the first drug is released during a first period of time and the second drug is released during a later second period of time. Dosage forms suitable for this type of release are generally considered targeted, enteric or timed-release dosage forms. Suitable dosage forms for this embodiment include, for example, a layered patch, layered or coated tablet, layered or coated osmotic device, capsule containing a mixture of beads that provide different release profiles for the drugs, and layered or coated implant.

Each drug will be released independently according to a rapid, immediate, controlled, sustained, extended, slow, timed, targeted, pseudo-first order, first order, pseudo-zero order, zero-order, and/or delayed release profile. The particular release profiles for the COX-II inhibitor and muscle relaxant in a particular dosage form will depend upon the specific COX-II inhibitor and muscle relaxant present. For example, a dosage form might provide: 1) a controlled release of the first drug and a controlled release of the second drug; 2) a controlled release of the second drug and a rapid release of the first drug; 3) a controlled release of the first drug and a rapid release of the second drug; 4) a rapid release of the first drug and the second drug; 5) a rapid release of the first drug and a delayed but rapid release of the second drug; 6) a rapid release of the first drug and a timed but controlled release of the second drug; 7) a rapid release of the second drug and a delayed but rapid release of the first drug, and 8) a rapid release of the second drug and timed but controlled release of the first drug.

A controlled release dosage form provides a controlled release of drug therefrom. Although not limited to any particular period of time, a controlled release dosage form generally releases drug over a period of about three hours up to about 12 hours, 16 hours, 18 hours, 20 hours, a day, several days, or a week, for example.

A delayed release dosage form is one that exhibits an initial delay in the release of drug after exposure to an environment of use. The period of delay is generally about 5 minutes to 12 hours, or 30 minutes to 10 hours, or 30 minutes to 8 hours or 30 minutes to 6 hours after administration.

A sustained release dosage form is one that provides a sustained release of drug over an extended period of time. The term "sustained release" is sometimes used synonymously with the terms "controlled release" or "extended release"; however, a sustained release dosage form generally provides a relatively constant rate of release for a period of time.

An immediate release dosage form is one that begins to release drug shortly, generally seconds to minutes, after exposure to an environment of use and therefore does not exhibit a significant delay in release of drug.

A timed release dosage form is one that begins to release drug after a predetermined period of time as measured from the moment of initial exposure to the environment of use.

A slow release dosage form is one that provides a slow rate of release of drug so that drug is released slowly and approximately continuously over a period of 3 hr, 6 hr, 12 hr, 18 hr, a day, 2 or more days, a week, or 2 or more weeks, for example.

A rapid release dosage form is one that releases drug over a period of 1-59 minutes or 0.1 to three hours once release has begun.

A targeted release dosage form generally refers to an oral dosage form that designed to deliver drug to a particular portion of the gastrointestinal tract of a subject. An exemplary targeted dosage form is an enteric dosage form that delivers a drug into the middle to lower intestinal tract but not into the stomach or mouth of the subject. Other targeted dosage forms can delivery to other sections of the gastrointestinal tract such as the stomach, jejunum, ileum, duodenum, cecum, large intestine, small intestine, colon, rectum,

A pseudo-first order release profile is one that approximates a first order release profile. A first order release profile characterizes the release profile of a dosage form that releases a constant percentage of an initial drug charge per unit time.

A pseudo-zero order release profile is one that approximates a zero-order release profile. A zero-order release profile characterizes the release profile of a dosage form that releases a constant amount of drug per unit time.

A delayed but rapid release dosage form is one that provides a delayed release of a drug followed by a rapid release of the drug. In other words, the beginning of the rapid release of drug is delayed by an initial period of time.

A delayed but controlled release dosage form is one that provides a delayed release of a drug followed by a controlled release of the drug. In other words, the beginning of the controlled release of drug is delayed by an initial period of time.

COX-II inhibitors useful in the present invention include those compounds that are selective for COX-II receptor inhibition over COX-I receptor inhibition or that are COX-II specific receptor inhibitors. These compounds include, for example, rofecoxib (VIOXX™, MK-0966), celecoxib (CELEBREX^{TM}, SC-58635), meloxicam, nimesulide, etodolac, flosulide (CGP-28238), NS-398, DUP-697, meloxicam, 6-methoxy-2-naphthylacetic acid (6-MNA), nabumetone (prodrug for 6-MNA), nimesulide, SC-5766, SC-58215, and T-14. Other suitable COX-II inhibitors are disclosed in PCT International Publications No. WO 99/25382, No. WO 94/15932, No. WO 96/03388, No. WO 95/00501, No. WO 95/18799, No. WO 98/50075, No. WO 99/13799 and No. WO 96/08482, the entire disclosures of which are hereby incorporated by reference. Still other suitable COX-II inhibitors are disclosed in Patents No. FR 2747123 or FR 2747124, the entire disclosures of which are hereby incorporated by reference. Additional suitable COX-II inhibitors are disclosed in US Patents No.5,393,790, No.5,409,944, No. 5,418,254, No. 5,420,343, No.5,436,265, No. 5,474,995, No. 5,476,944, No. 5,486,534, No. 5,510,368, No. 5,521,213, No. 5,536,752, No. 5,547,975, No. 5,550,142, No. 5,552,422, No. 5,565,482, No. 5,576,339, No. 5,580,985, No. 5,585,504, No. 5,593,994, No. 5,616,601, No. 5,604,260, No. 5,639,780, No. 5,604,253, No. 5,130,311 and No. 5,596,008, the entire disclosures of which are hereby incorporated by reference.

Useful cyclooxygenase-2 inhibitors also include the substituted spiro compounds of U.S. Patent No. 5,393,790, e.g., 5-(4-fluorophenyl)-6-[4-(methylsulfonyl)phenyl]-spiro[2,4]hept-5-ene, 4-[6-(4-fluorophenyl)spiro[2,4]hept-5-en-5-yl]benzenesulfonamide-6(4-fluorophenyl)-7-[4-(methylsulfonyl)phenyl]spiro[3.4]oct-6-ene, and others known to those of ordinary skill in the art; the sulfonamides of U.S. Patent No. 5,409,944,e.g., 5-methanesulfonamido-6-(2-(4-methyl- 1 ,3-diazinylthio-thienylthio))- 1 -indanone, 5-methanesulfonamido-6(2-thiazolythio)-1-indanone, and others known to those of ordinary skill in the art; the 2,3 substituted cyclopentadienyl compounds of U.S. Patent No. 5,418,254, e.g., 1 -methylsulfonyl-4-[1,1-dimethyl-4-(4-fluorophenyl)cyclopenta-2,4-dien-3-yl]benzene, 4-[4-(4- fluorophenyl)-1,1dimethylcyclopenta-2,4-dien-3-yl]benzene-sulfonamide, 1 -methylsulfonyl-4 {4-(4-trifluoromethylphenyl)-1 -trifluoromethyl-cyclopenta-2,4-dien-3-yl]benzene, and others known to those of ordinary skill in the art; the aromatic cycloethers of U.S. Patent No. 5,420,343, e.g., methyl 3,5-bis(1, dimethylethyl)benzoate, 3,5-bis(1,1-dimethylethyl) benzenemethanol, 1,3-bis(1,1-dimethylethyl)-5(2-chloroethyl)benzene, and others known to those of ordinary skill in the art; the 1-aroyl acids of U.S. Patent No. 5,436,265, e.g., 1-(2,4,6-trichlorobenzoyl)-5-methoxy-2-methyl-3-indolyl acetic acid, 1-(2,6-dichlorobenzoyl)-5-methoxy-2-methyl-3-indolyl acetic acid and others known to those of ordinary skill in the art; the phenyl heterocycles of U.S. Patent Nos. 5,474,995, 5,536,752 and 5,550,142, e.g. 3-(4-(aminosulfonyl)phenyl)-2-(4-fluorophenyl)thiophene, 2-(4-fluorophenyl)-3-(4-(methylsulfonyl)phenyl)-2-cyclopentenone, 4-(4-methylsulfonyl)phenyl)isothiazole, and others known to those of ordinary skill in the art; the cyclic phenolic thioether derivatives of U.S. Patent No. 5,476,944, e.g., 3,5-bis(1,1-dimethylethyl)benzenethiol, trans-2-[[3,5-bis( 1,1 dimethylethyl)henyl]hio]cyclohexanol, 3,6-dioxabicyclo-[3.1.0]hexane, and others known to those of ordinary skill in the art; the 3,4-substituted pyrazoles of U.S. Patent No. 5,486,534, e.g., 4-(4-fluorophenyl)-1-methyl-3-[4-(methylsulfonyl)phenyl]-5-trifluoromethyl)pyrazole, 1-benzyl-4-(4-fluorophenyl)-3-[4-methylsulfonyl)phenyl-5-(trifluoromethyl)pyrazole, 1-allyl-4(4-fluorophenyl)-3-[4-methylsulfonyl)pheyl]-5-(trifluoromethyl)-1H-pyrazole, and others known to those of ordinary skill in the art; the N-benzyl-3-indoleacetic acids of U.S Patent No. 5,510,368, e.g., 2-(5-bromo-1(4-bromobenzyl)-2-methyl-1H-indol-3-yl)propionic acide, (S)-(+)-2-(5-bromophenyl)-2-methyl-1H-indol-3-yl)acetyl acid, (R)-(-)-2-(5-bromo-1(4-bromobenzyl)-2-methyl-1H-indol-3-yl)propionic acid, and others known to those of ordinary skill in the art; the diaryl bicyclic heterocyclics of U.S. Patent No. 5,521,213, e.g., 3-(4-(methylsulfonyl)phenyl)-2-phenylbenzo[b]furan, 3-(4-(methanesulfonyl)phenyl)-2-phenylbenzo[b]thiophene, 2-(4-fluorophenyl)-3-(4-amino sulfonyl)pheyl)-4H-thieno[2,3-c]furan-6-one, and others known to those of ordinary skill in the art; thebenzopyranopyrazolyl derivatives of U.S. Patent No. 5,547,975, e.g., 4-[1,4-dihydro-3-(trifluoromethyl)-[1]benzopyranol[4,3-c]pyrazol- 1-yl]benzensulfonamide, methyl[1-[4-(aminosulfonyl)phenyl]-1,4-dihydro-[1]benzopyranol[4,3-c]pyrazol-3-yl] carboxylate, 4[3-(trifluoromethyl)-1H-benzofuro[3,2-c]pyrazol-1-yl]benzenesulfonamide, and others known to those of ordinary skill in the art; the aryl substituted 5,5 fused aromatic nitrogen compounds of U.S. Patent No. 5,552,42, e.g., 5-(4-methylsulfonyl)phenyl)-6-phenylimidazo[2m1-b]thiazole, 2-methyl-5-(methylsulfonyl)pheyl)-6-phenylimidazo[2,1-b]thiazole, 3-methyl-5(4-methylsulfonyl)phenyl)-6-phenylimidazo[2,1-b]thiazole, and others known to those of ordinary skill in the art; the heteroarylpyranopyrazolyl derivatives of U.S. Patent No. 5,565,482, e.g., 4-[1,5-dihydro-6-fluoro-7-methoxy-3-(trifluoromethyl)-[2]benzothiopyranol[4,3-c]pyrazol, 4-[1,4-dihydro-3-(trifluoromethyl)-[1]benzopyrano[4,3-c]pyrazol-1-yl]benzenesulfonamide, 1,5-dihydro-6-fluoro-7-methoxy-1-[(4-methylsulfonyl)phenyl]-3-(trifluoromethyl)-[2]benzothiopyrano-[4,3-c]pyrazol-1-yl]benzenesulfonamide, and others known to those of ordinary skill in the art; the pyridyl substituted cyclopentadienes of U.S. Patent No.5,576,339, e.g., 1-methylsulfonyl-4-[1,1-dimethyl-4-(4-fluorophenyl)cyclopenta-2,4-dien-3-yl]benzene, 4-[4-(4-fluoropyhenyl0-1,1-dimethylcyclopenta-2,4-dien-3-yl]benzenesulfonamide, and others known to those of ordinary skill in the art; the substituted pyrazoles of U.S. Patent No. 5,580,985, e.g., 1-ethyl-4-(4fluorophenyl)-3-[4-fluorophenyl)-1-[4-(methylthio)phenyl]-2-buten-1-one, 1-benzyl-4-(fluorophenyl)-3-[4-(methylsulfonyl)phenyl]-5-(trifluoromethyl)pyrazole, and others known to those of ordinary skill in the art; the lactones of U.S. Patent No. 5,585,504, e.g., 3-phenyl-4-(4-methylsulfonyl)phenyl-2-(5H)-furanone, 3-(3,4-difluorophenyl)-4-(4-methylsulfonyl)phenyl)-2-(5H)-furanone, and others known to those of ordinary skill in the art; the ortho substituted phenyl compounds of U.S. Patent No. 5,593,994, e.g., 2-[(4-methylthio)phenyl]-1-biphenyl,1-cyclohexene-2(4'-methylsulfonylphenyl)benzene, 3-(4'-methylsulfonylphenyl)-4-phenylphenol, and others known to those of ordinary skill in the art; and the 3,4-diaryl substituted pyridines of U.S. Patent No. 5,596,008, e.g., 5-(4-fluorophenyl)-2-methoxy-4-[4-(methylsulfonyl)phenyl]-6-(trifluoromethyl)pyridine, 2-ethoxy-5-(4-fluorophenyl)-4-[4-(methylsulfonyl)phenyl]-6-(trifluoromethyl)-pyridine, 5-(4-fluorophenyl)-4[4-methylsulfonyl)phenyl]-2-(2-propynyloxy)-6-(trifluorophenyl)-6-(trifluoromethyl)pyridine, and others known to those of ordinary skill in the art.

A muscle relaxant is a compound which pharmacological profile includes at least a significant, or major, muscle relaxing effect. Muscle relaxants useful in the present invention include, for example, alcuronium, aminophylline, baclofen, carisoprodol (SOMA®), chlorphenesin, chlorphenesin carbamate, chlorzoxazone (PARAFON FORTE®), cyclobenzaprine (FLEXERIL®), dantrolene, decamethonium, diazepam, dyphylline, ethaverine, gallamine triethiodide, hexafluorenium, mephenesin, metaxalone (SKELAXIN®), methocarbamol (ROBAXIN®), metocurine iodide, orphenadrine (NORFLEX®), pancuronium, papaverine, pipecuronium, pridinol (pridinolum), succinylcholine, theophylline, tubocurarine, vecuronium, idrocilamide, ligustilide, cnidilide, senkyunolide, and others known to those of ordinary skill in the pharmaceutical sciences.

The COX-II inhibitor and muscle relaxant are independently used in their free acid, free base or pharmaceutically acceptable salt forms. When mentioned herein, the COX-II inhibitor and muscle relaxant are taken be independently present in their free and/or salt forms. As used herein, "pharmaceutically acceptable salts" refer to derivatives of the disclosed compounds wherein the therapeutic compound is modified by making acid or base salts thereof. Examples of pharmaceutically acceptable salts include, but are not limited to, mineral or organic acid salts of the COX-II inhibitor or muscle relaxant. The pharmaceutically acceptable salts include the conventional non-toxic salts, for example, from non-toxic inorganic or organic acids. For example, such conventional non-toxic salts include those derived from inorganic acids such as hydrochloric, hydrobromic, sulfuric, sulfonic, sulfamic, phosphoric, nitric and others known to those of ordinary skill in the art; and the salts prepared from organic acids such as amino acids, acetic, propionic, succinic, glycolic, stearic, lactic, malic, tartaric, citric, ascorbic, pamoic, maleic, hydroxymaleic, phenylacetic, glutamic, benzoic, salicylic, sulfanilic, 2-acetoxybenzoic, fumaric, toluenesulfonic, methanesulfonic, ethane disulfonic, oxalic, isethionic, and other known to those of ordinary skill in the pharmaceutical sciences. For acidic compounds, the salt may include an amine-based (primary, secondary, tertiary or quaternary amine) counter ion, an alkali metal cation, or a metal cation. Lists of suitable salts are found in texts such as *Remington's Pharmaceutical Sciences,* 18th Ed. (Alfonso R. Gennaro, ed.; Mack Publishing Company, Easton, PA, 1990); *Remington: the Science and Practice of Pharmacy* 19^{th} Ed.( Lippincott, Williams & Wilkins, 1995); *Handbook of Pharmaceutical Excipients,* 3^{rd} Ed. (Arthur H. Kibbe, ed.; Amer. Pharmaceutical Assoc., 1999); the *Pharmaceutical Codex: Principles and Practice of Pharmaceutics* 12^{th} Ed. (Walter Lund ed.; Pharmaceutical Press, London, 1994); The United States Pharmacopeia: The National Formulary (United States Pharmacopeial Convention); and *Goodman and Gilman's: the Pharmacological Basis of Therapeutics* (Louis S. Goodman and Lee E. Limbird, eds.; McGraw Hill, 1992), the disclosures of which are hereby incorporated by reference.

The phrase "pharmaceutically acceptable" is employed herein to refer to those compounds, materials, compositions, and/or dosage forms which are, within the scope of sound medical judgment, suitable for use in contact with the tissues of human beings and animals without excessive toxicity, irritation, allergic response, or other problem or complication, commensurate with a reasonable benefit/risk ratio.

The pharmaceutical composition of the invention can be included in any dosage form. The dosage form may be adapted for oral, buccal, sublingual, optic, otic, rectal, vaginal, topical, nasal, urethral or parental delivery. Suitable dosage forms include, for example, tablet, capsule, bead, granule, sphere, paste, pastille, pill, prill, suspension, osmotic device, powder, liquid, gelcap, troche, stick, suppository, implant, lollipop, spray, inhaler or patch. Alternatively, the pharmaceutical composition may be included in a candy or food product.

The pharmaceutical formulation of the invention can be included in an osmotic device. Such osmotic devices can be made according to U.S. Patent No. 4,014,334 to Theeuwes et al., U.S. Patent No. 4,576,604 to Guittard et al., Argentina Patent No. 234,493, U.S. Patent No. 4,673,405 to Guittard et al., U.S. Patent No. 5,558,879 to Chen et al., U.S. Patent No. 4,810,502 to Ayer et al., U.S. Patent No. 4,801,461 to Hamel et al., U.S. Patent No. 5,681,584 to Savastano et al., U.S. Patent No. 3,845,770, U.S. Patent No. 4,008,719 to Theeuwes et al., U.S. Patent No. 4,058,122 to Theeuwes et al., U.S. Patent No. 4,116,241 to Theeuwes et al., U.S. Patent No. 4,160,452 to Theeuwes, U.S. Patent No. 4,256,108 to Theeuwes, nd Argentina Patent No. 199,301, the entire disclosures of which are hereby incorporated by reference, and other osmotic device formulations known to those of ordinary skill in the art.

Osmotic devices such as those described by Faour et al. (U.S. 6,004,582), the entire disclosure of which is hereby incorporated by reference, are particularly advantageous for delivering two different drugs from a single osmotic device tablet. Faour et al. disclose osmotic device formulations comprising a slow release drug-containing core combined with a rapid release coating.

Controlled release formulations containing the pharmaceutical formulation of the invention can be made according to Biorelated Polymers and Gels: Controlled Release and Applications in Biomedical Engineering (ed. Teruo Okano; 1998); Encyclopedia of Controlled Drug Delivery (ed. Edith Mathiowitz; 1999); Future Strategies for Drug Delivery with Particulate Systems (ed. J.E. Diederichs; 1998); Controlled Release Series (ed. J.M. Anderson; 1987); Controlled Drug Delivery Series (Ed. S.D. Bruck; 1983); Controlled Release of Drugs Series (ed. M. Rosoff; 1989); Controlled Release Technology: Pharmaceutical Applications (ACS Symposium Series No. 348) (eds. P.I. Lee and W.R. Good; 1987); Extended Release Dosage Forms (ed. L. Krowczynski; 1987); Handbook of Pharmaceutical Controlled Release Technology (ed. D.L. Wise; 2000); Intelligent Materials for Controlled Release (ed. S.M. Dinh; 1999); Multicomponent Transport in Polymer Systems for Controlled Release (Polymer Science and Engineering Monograph Series) (ed. A. Polishchuk; 1997); Pharmaceutical Technology: Controlled Drug Release (ed. M. Rubenstein; 1987); Polymers for Controlled Drug Delivery (ed. P.J. Tarcha; 1991); Tailored Polymeric Materials for Controlled Delivery Systems (ACS Symposium Series No. 709) (ed. I. McCulloch; 1998); Oral Colon-Specific Drug Delivery (ed. D.R. Friend, 1992); and other publications known to those of ordinary skill in the art, the entire disclosures of which are hereby incorporated by reference

Topical formulations for administering the pharmaceutical formulation of the invention can be prepared as disclosed in Electrically Assisted Transdermal and Topical Drug Delivery (ed. A.K. Banga; 1998); Topical Drug Bioavailability, Bioequivalence and Penetration (ed. V.P. Shah; 1993); Topical Drug Delivery Formulations (ed. D.W. Osborne); Transdermal and Topical Drug Delivery Systems (ed. T.K. Ghosh; 1997); and other publications known to those of ordinary skill in the art, the entire disclosures of which are hereby incorporated by reference.

The pharmaceutical composition of the invention can also be administered in other dosage forms such as those disclosed in Handbook on Injectable Drugs 3rd Ed. (Trissel, 1983); Wang, et al., "Review of Excipients and pH's for Parenteral Products Used in the United States", *Journal of the Parenteral Drug Association* 14(6):452 (1980) and Hard Capsules Development and Technology (The Pharmaceutical Press, 1987).

The dissolution profile for the formulation of Example 1 is generally described as follows. The dissolution profile depicted in FIG. 1 indicates that the pridinol is released completely and rapidly over a period of less than one hour, less than 45 min, less than 30 min, or less than 15 min. The dissolution profile depicted in FIG. 2 indicates that the rofecoxib is released completely over a period of less than two hours. In addition, at least 80% of the rofecoxib is released in less than 60 min, less than 45 min, less than 30 min or less than 20 min.

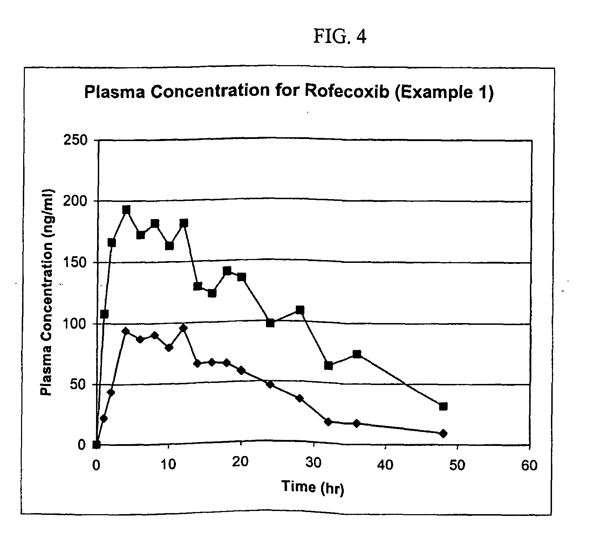

The tablet formulation of Example 1 provides therapeutically effective levels of the COX-II inhibitor and muscle relaxant for at least a predetermined period of time, generally not less than 18 hours and not more than 60 hours, not less than 20 hours and not more than 48 hours, or not less than 22 hours and not more than 48 hours. FIG. 3 depicts the mean plasma concentration for rofecoxib as provided by the rapid release tablet of Example 1. FIG. 4 depicts the generally observed maximum and minimum plasma concentrations for rofecoxib as provided by the rapid release tablet of Example 1. The plasma concentration versus time profile for rofecoxib in the formulation of Example 1 is generally described as follows.

| **Time after Administration (hr)** | **Mean Plasma Concentration (ng/ml)** | **Standard Deviation (ng/ml)** | **Minimum Plasma Concentration (ng/ml)** | **Maximum Plasma Concentration (ng/ml)** |
|---|---|---|---|---|
| 0 | 0 | 0 | 0 | 0 |
| 1 | 55.8 | 30.2 | 20.8 | 108 |
| 2 | 102.2 | 38.3 | 43.5 | 166.3 |
| 4 | 148.2 | 35.4 | 94.2 | 193.2 |
| 6 | 116 | 26.5 | 86.8 | 172.4 |
| 8 | 134 | 28.1 | 90.4 | 181.9 |
| 10 | 123.5 | 27.5 | 79.3 | 163.2 |
| 12 | 126.2 | 28.2 | 95.8 | 181.8 |
| 14 | 96.2 | 24.1 | 65.3 | 129.4 |
| 16 | 93.9 | 19.9 | 65.9 | 123.2 |
| 18 | 94.5 | 22.9 | 65 | 141.3 |
| 20 | 89.1 | 23.8 | 58.4 | 136 |
| 24 | 69.2 | 16.1 | 46.8 | 97.7 |
| 28 | 62.7 | 21.6 | 35.3 | 108.6 |
| 32 | 39.3 | 12.9 | 16.5 | 63.6 |
| 36 | 35.6 | 17.1 | 16.3 | 73.9 |
| 48 | 16.6 | 6.6 | 8.8 | 31.3 |

The actual plasma concentration profile observed for the COX-II inhibitor and muscle relaxant will depend upon the dosage form used to administer the drugs, the amounts in which the drugs are present in the dosage form, the specific drugs used and the behavior of the dosage in a being to which it is administered.

Alternatively, the plasma concentration profile for rofecoxib as provided after administration of a single tablet made according to Example 1 can be described as follows.

| **Time after Administration (hr)** | **Plasma Concentration (ng/ml)** |
|---|---|
| 0 | 0 |
| 1 | 20-80 |
| 2 | 60-140 |
| 4 | 110-180 |
| 6 | 90-140 |
| 8 | 105-165 |
| 10 | 95-150 |
| 12 | 100-150 |
| 14 | 70-125 |
| 16 | 70-110 |
| 18 | 70-110 |
| 24 | 55-85 |
| 32 | 25-55 |
| 48 | 10-20 |

The osmotic device of the present invention can comprise a drug-containing water soluble coating present in an amount of about 9-40% wt., at least about 25% wt., about 25-40% wt. and about 30-40% wt. based upon the total weight of the osmotic device. The higher weight ranges are remarkable, since no prior art osmotic devices are known that include a sprayed drug-containing water soluble coating present in such high amounts. Also, the core is present in an amount of about 50-80% wt., about 50-75% wt., about 50-65% wt., or about 54-63% wt. based upon the total weight of the device.

Those of ordinary skill in the art will appreciate that the particular amounts of drug used in the pharmaceutical formulation will vary according to, among other things, the desired pharmacokinetic or pharmacological behavior in a mammal.

A water soluble or erodible coat, or layer, which is either inert or drug-containing, will generally comprise an inert and non-toxic material which is at least partially, and optionally substantially completely, soluble or erodible in an environment of use. Selection of suitable materials will depend upon the desired behavior of the dosage form. A rapidly dissolving coat or layer will be soluble in the buccal cavity and/or upper GI tract, such as the stomach, duodenum, jejunum or upper small intestines. Exemplary materials are disclosed in U.S. Patents No. 4,576,604 to Guittard et al. and No. 4,673,405 to Guittard et al., and No. 6,004,582 to Faour et al. and the text *Pharmaceutical Dosage Forms: Tablets Volume I,* 2^{nd} *Edition.* (A. Lieberman. ed. 1989, Marcel Dekker, Inc.), the relevant disclosures of which are hereby incorporated by reference. In some embodiments, the rapidly dissolving coat or layer will be soluble in saliva, gastric juices, or acidic fluids.

Materials which are suitable for making the water soluble or erodible coat or layer include, by way of example and without limitation, water soluble polysaccharide gums such as carrageenan, fucoidan, gum ghatti, tragacanth, arabinogalactan, pectin, and xanthan; water-soluble salts of polysaccharide gums such as sodium alginate, sodium tragacanthin, and sodium gum ghattate; water-soluble hydroxyalkylcellulose wherein the alkyl member is straight or branched of 1 to 7 carbons such as hydroxymethylcellulose, hydroxyethylcellulose, and hydroxypropylcellulose; synthetic water-soluble cellulose-based lamina formers such as methyl cellulose and its hydroxyalkyl methylcellulose cellulose derivatives such as a member selected from the group consisting of hydroxyethyl methylcellulose, hydroxypropyl methylcellulose, and hydroxybutyl methylcellulose; croscarmellose sodium; other cellulose polymers such as sodium carboxymethylcellulose; and other materials known to those of ordinary skill in the art. Other lamina forming materials that can be used for this purpose include poly(vinylpyrrolidone), polyvinylalcohol, polyethylene oxide, a blend of gelatin and polyvinyl-pyrrolidone, gelatin, glucose, saccharides, povidone, copovidone, poly(vinylpyrrolidone)-poly(vinyl acetate) copolymer. The water soluble coating can comprise other pharmaceutical excipients that do or do not alter the way in which the water soluble coating behaves. The artisan of ordinary skill will recognize that the above-noted materials include film forming polymers.

Other materials include hydroxypropylcellulose, microcrystalline cellulose (MCC, Avicel.TM. from FMC Corp.), poly(ethylene-vinyl acetate) (60:40) copolymer (EVAC from Aldrich Chemical Co.), 2-hydroxyethylmethacrylate (HEMA), MMA, terpolymers of HEMA:MMA:MA synthesized in the presence of N,N'-bis(methacryloyloxyethyloxycarbonylamino)-azobenzene, azopolymers, enteric coated timed release system (Time Clock® from Pharmaceutical Profiles, Ltd., UK) and calcium pectinate can be included in the water soluble coat.

The inert water soluble or erodible coat covering the semipermeable wall and blocking the passageway is made of synthetic or natural material which, through selective dissolution or erosion shall allow the passageway to be unblocked thus allowing the process of osmotic delivery to start. This slow or fast dissolving or eroding coat can be impermeable to a first external fluid, while being soluble or erodible in a second external fluid. This property can help to achieve a controlled and selective release of the active compound in the nucleus.

In some embodiments, a coat or layer of a dosage form will be insoluble in the fluid of a first environment of use, such as gastric juices, acidic fluids, or polar liquids, and soluble or erodible in the fluid of a second environment of use, such as intestinal juices, substantially pH neutral or basic fluids, or apolar liquids. A wide variety of other polymeric materials are known to possess these various solubility properties and can be included in the coat or layer. Such other polymeric materials include, by way of example and without limitation, cellulose acetate phthalate (CAP), cellulose acetate trimelletate (CAT), poly(vinyl acetate)phthalate (PVAP), hydroxypropyl methylcellulose phthalate (HP), poly(methacrylate ethylacrylate) (1:1) copolymer (MA-EA), poly(methacrylate methylmethacrylate) (1:1) copolymer (MA-MMA), poly(methacrylate methylmethacrylate) (1:2) copolymer, Eudragit^{TM} L-30-D (MA-EA, 1:1), Eudragit^{TM} L-100-55 (MA-EA, 1:1), hydroxypropyl methylcellulose acetate succinate (HPMCAS), Coateric^{TM} (PVAP), Aquateric^{TM} (CAP), AQOAT^{TM} (HPMCAS) and combinations thereof. The water soluble or erodible coat can also comprise dissolution aids, stability modifiers, and bioabsorption enhancers.

An optional polymeric material for use in a coated osmotic device, tablet or bead includes enteric materials that resist the action of gastric fluid. A material that easily adapts to this kind of requirement is a poly(vinylpyrrolidone)-vinyl acetate copolymer, such as the material supplied by BASF under its Kollidon VA64 trademark, mixed with magnesium stearate and other similar excipients.

A water soluble coat or layer can also comprise povidone, which is supplied by BASF under its Kollidon K 30 trademark, and hydroxypropyl methylcellulose, which is supplied by Dow under its Methocel E-15 trademark. The materials can be prepared in solutions having different concentrations of polymer according to the desired solution viscosity. For example, a 10% P/V aqueous solution of Kollidon™ K 30 has a viscosity of about 5.5-8.5 cps at 20° C., and a 2% P/V aqueous solution of Methocel™ E-15 has a viscosity of about 13-18 cps at 20° C.

A water soluble coat or layer can also comprise other materials suitable which are substantially resistant to gastric juices and which will promote either enteric or colonic release. For this purpose, the inert water soluble coat can comprise one or more materials that do not dissolve, disintegrate, or change their structure in the stomach and during the period of time that a dosage form resides in the stomach. Representative materials that keep their integrity in the stomach can comprise a member selected from the group consisting of (a) keratin, keratin sandarac-tolu, salol (phenyl salicylate), salol betanaphthylbenzoate and acetotannin, salol with balsam of Peru, salol with tolu, salol with gum mastic, salol and stearic acid, and salol and shellac; (b) a member selected from the group consisting of formalized protein, formalized gelatin, and formalized cross-linked gelatin and exchange resins; (c) a member selected from the group consisting of myristic acid-hydrogenated castor oil-cholesterol, stearic acid-mutton tallow, stearic acid-balsam of tolu, and stearic acid-castor oil; (d) a member selected from the group consisting of shellac, ammoniated shellac, ammoniated shellac-salol, shellac-wool fat, shellac-acetyl alcohol, shellac-stearic acid-balsam of tolu, and shellac n-butyl stearate; (e) a member selected from the group consisting of abietic acid, methyl abictate, benzoin, balsam of tolu, sandarac, mastic with tolu, and mastic with tolu, and mastic with acetyl alcohol; (f) acrylic resins represented by anionic polymers synthesized from methacrylate acid and methacrylic acid methyl ester, copolymeric acrylic resins of methacrylic and methacrylic acid and methacrylic acid alkyl esters, copolymers of alkacrylic acid and alkacrylic acid alkyl esters, acrylic resins such as dimethylaminoethylmethacrylate-butylmethacrylate-methylmethacrylate copolymer of 150,000 molecular weight, methacrylic acid-methylmethacrylate 50:50 copolymer of 135,000 molecular weight, methacrylic acid-methylmethacrylate-30:70-copolymer of 135,000 mol. wt., methacrylic acid-dimethylaminoethyl-methacrylate-ethylacrylate of 750,000 mol. wt., methacrylic acid-methylmethacrylate-ethylacrylate of 1,000,000 mol. wt., and ethylacrylate-methylmethacrylate-ethylacrylate of 550,000 mol. wt; and, (g) an enteric composition comprising a member selected from the group consisting of cellulose acetyl phthalate, cellulose diacetyl phthalate, cellulose triacetyl phthalate, cellulose acetate phthalate, hydroxypropylmethylcellulose phthalate, sodium cellulose acetate phthalate, cellulose ester phthalate, cellulose ether phthalate, methylcellulose phthalate, cellulose ester-ether phthalate, hydroxypropyl cellulose phthalate, alkali salts of cellulose acetate phthalate, alkaline earth salts of cellulose acetate phthalate, calcium salt of cellulose acetate phthalate, ammonium salt of hydroxypropyl methylcellulose phthalate, cellulose acetate hexahydrophthalate, hydroxypropyl methylcellulose hexahydrophthalate, polyvinyl acetate phthalate diethyl phthalate, dibutyl phthalate, dialkyl phthalate wherein the alkyl comprises from 1 to 7 straight and branched alkyl groups, aryl phthalates, and other materials known to one or ordinary skill in the art.

A semipermeable membrane included in an osmotic device dosage form is generally formed of a material that is substantially permeable to the passage of fluid from the environment of use to the core and substantially impermeable to the passage of active agent from the core. Many common materials that form a semipermeable wall which are known by those of ordinary skill in the art of pharmaceutical sciences are suitable for this purpose. Exemplary materials are cellulose esters, cellulose ethers and/or cellulose ester-ethers. These materials can be mixed with any of the plasticizers disclosed herein. However, it has been found that a semipermeable membrane comprising cellulose acetate (CA) and optionally poly(ethylene glycol) (PEG), in particular PEG 400, performs well when used in combination with the other materials required in the present osmotic device. This particular combination of CA and PEG provides a semipermeable membrane that gives the osmotic device a well controlled release profile for the active agent in the core and that retains its chemical and physical integrity in the environment of use. The ratio of CA:PEG generally ranges from about 50-99% by weight of CA: about 50-1% by weight of PEG, and about 95% by weight of CA: about 5% by weight of PEG. The ratio can be varied to alter permeability and ultimately the release profile of the osmotic device. Other suitable materials can include a selected member of the group of cellulose acylates such as cellulose acetate, cellulose diacetate, cellulose triacetate and combinations thereof. Many suitable polymers, include those disclosed in Argentine Patent No. 199,301, U.S. Patent No. 6004,582 and references cited herein, the disclosures of which are hereby incorporated by reference.

Representative materials for the semipermeable membrane include a member selected from the group consisting of cellulose acylate, cellulose diacylate, cellulose triacylate, cellulose acetate, cellulose diacetate, cellulose triacetate, mono, di- and tricellulose alkanylates, mono-, di- and tricellulose aroylates, and others known to those of ordinary skill in the art. Exemplary polymers include cellulose acetate having a D.S. up to 1 and an acetyl content up to 21%; cellulose acetate having an acetyl content of 32 to 39.8%; cellulose diacetate having a D.S. of 1 to 2 and an acetyl content of 21 to 35%; cellulose triacetate having a D.S. of 2 to 3 and an acetyl content of 35 to 44.8%; and others known to those of ordinary skill in the art. More specific cellulosic polymers include cellulose propionate having a D.S. of 1.8 and a propionyl content of 39.2 to 45% and a hydroxyl content of 2.8 to 5.4%; cellulose acetate butyrate having a D.S. of 1.8, an acetyl content of 13 to 15% and a butyryl content of 34 to 39%; cellulose acetate butyrate having an acetyl content of 2 to 29%; a butyryl content of 17 to 53% and a hydroxyl content of 0.5 to 4.7%; cellulose triacylates having a D.S. of 2.9 to 3 such as cellulose trivalerate, cellulose trilaurate, cellulose tripalmitate, cellulose trisuccinate, and cellulose trioclanoate; cellulose diacylates having a D.S. of 2.2 to 2.6 such as cellulose disuccinate, cellulose dipalmitate, cellulose dioclanoate, cellulose dipentale, and others known to those of ordinary skill in the art. Additional semipermeable polymers include acetaldehyde dimethyl acetate, cellulose acetate ethyl carbamate, cellulose acetate phthalate for use in environments having a low ph, cellulose acetate methyl carbamate, cellulose acetate dimethyl aminoacetate, semipermeable polyamides, semipermeable polyurethanes, semipermeable sulfonated polystyrenes, cross-linked selectively semipermeable polymers formed by the coprecipitation of a polyanion and a polycation as disclosed in U.S. Patents No. 3,173,876, No. 3,276,586, No. 3,541,005, No.3,541,006, and No. 3,546,142; semipermeable polymers as disclosed by Loeb and Sourirajan in U.S. Pat. No. 3,133,132; lightly cross-linked polystyrene derivatives; cross-linked poly(sodium styrene sulfonate), cross-linked poly(vinylbenzyltrimethyl ammonium chloride), semipermeable polymers exhibiting a fluid permeability of 10.sup.-5 to 10.sup.-1 (cc.mil/cm.sup.2.hr.atm) expressed as per atmosphere of hydrostatic or osmotic pressure difference across the semipermeable wall. These and others polymers are disclosed in U.S. Patents No. 3,845,770, No. 3,916,899, No. 4,765,989 and No. 4,160,020; and in *Handbook of Common Polymers* (Scott, J. R. and Roff, W. J., eds.; 1971; CRC Press, Cleveland, Ohio).

When the pharmaceutical composition is contained within an osmotic device dosage form, the osmotic device of the invention comprises at least one passageway (pore, hole, or aperture) which communicates the exterior of the semipermeable wall with the core of the device. The passageway can be formed according to any of the known methods of forming passageways in a semipermeable membrane. Such methods include, for example, 1) drilling a hole through the semipermeable membrane with a bit or laser; 2) including a water soluble material within the composition that forms the semipermeable membrane such that a pore forms when the osmotic device is in an aqueous environment of use; 3) punching a hole through the semipermeable membrane; or 4) employing a tablet punch having a pin to punch a hole through the semipermeable lamina. The passageway can pass through the semipermeable wall and one or more of any other lamina coated onto the semipermeable membrane or between the semipermeable membrane and the core. The passageway(s) can be shaped as desired. In some embodiments, the passageway is laser drilled and is shaped as an oval, ellipse, slot, slit, cross or circle.

Methods of forming passageways in semipermeable membranes of osmotic devices are disclosed in U.S. Patents No. 4,088,864 to Theeuwes et al., No. 4,016,880 to Theeuwes et al., No. 3,916,899 to Theeuwes et al., No. 4,285,987 to Ayer et al., No. 4,783,337 to Wong et al., No. 5,558,879 to Chen et al., No. 4,801,461 to Hamel et al., and No. 3,845,770 to Theeuwes et al., the disclosures of which are hereby incorporated by reference.

The core of an osmotic device, bead or tablet dosage form of the present invention will comprise a COX-II inhibitor and/or muscle relaxant, at least one pharmaceutically acceptable excipient and optionally one or more other materials. Generally, the tablet formulations will comprise about 0.1-99.9% by weight of drug in the uncoated tablet core. Acceptable ranges may vary according to the desired therapeutic response, the tablet size, the amount and type of excipients used in the core of the device, the combination of drugs used and the intended use of the osmotic device.

When the controlled release tablet is an osmotic device, osmotically effective solutes, osmotic agents or osmagents are added. These osmagents can aid in either the suspension or dissolution of the drug in the core. Exemplary osmagents include organic and inorganic compounds such as salts, acids, bases, chelating agents, sodium chloride, lithium chloride, magnesium chloride, magnesium sulfate, lithium sulfate, potassium chloride, sodium sulfite, calcium bicarbonate, sodium sulfate, calcium sulfate, calcium lactate, d-mannitol, urea, tartaric acid, raffinose, sucrose, alpha-d-lactose monohydrate, glucose, combinations thereof and other similar or equivalent materials which are widely known in the art. Osmagents can also be incorporated to the core of the osmotic device to control the release of drug therefrom. U.S. Patent No. 4,077,407 to Theeuwes et al., the entire disclosure of which is hereby incorporated by reference, discloses suitable osmagents.

The dosage forms of the invention can also comprise an acidifying agent, adsorbents, alkalizing agent, antioxidants, buffering agent, colorant, flavorant, sweetening agent, antiadherent, binder, diluent, direct compression excipient, disintegrant, glidant, lubricant, opaquant, polishing agent, complexing agents, fragrances, preservative and combinations thereof.

A used herein, a complexing agent is an agent that complexes metal ions. Exemplary complexing agents include EDTA disodium, edetate, pentates and others known to those of ordinary skill in the art.

As used herein, the term "acidifying agent" is intended to mean a compound used to provide an acidic medium for product stability. Such compounds include, by way of example and without limitation, acetic acid, amino acid, citric acid, fumaric acid and other alpha hydroxy acids, such as hydrochloric acid, ascorbic acid, and nitric acid and others known to those of ordinary skill in the art.

As used herein, the term "adsorbent" is intended to mean an agent capable of holding other molecules onto its surface by physical or chemical (chemisorption) means. Such compounds include, by way of example and without limitation, powdered and activated charcoal and other materials known to one of ordinary skill in the art.

As used herein, the term "alkalizing agent" is intended to mean a compound used to provide alkaline medium for product stability. Such compounds include, by way of example and without limitation, ammonia solution, ammonium carbonate, diethanolamine, monoethanolamine, potassium hydroxide, sodium borate, sodium carbonate, sodium bicarbonate, sodium hydroxide, triethanolamine, and trolamine and others known to those of ordinary skill in the art.

As used herein, the term "antioxidant" is intended to mean an agent which inhibits oxidation and thus is used to prevent the deterioration of preparations by the oxidative process. Such compounds include, by way of example and without limitation, ascorbic acid, ascorbyl palmitate, butylated hydroxyanisole, butylated hydroxytoluene, hypophophorous acid, monothioglycerol, propyl gallate, sodium ascorbate, sodium bisulfite, sodium formaldehyde sulfoxylate and sodium metabisulfite and other materials known to one of ordinary skill in the art.

As used herein, the term "buffering agent" is intended to mean a compound used to resist change in pH upon dilution or addition of acid or alkali. Such compounds include, by way of example and without limitation, potassium metaphosphate, potassium phosphate, monobasic sodium acetate and sodium citrate anhydrous and dihydrate and other materials known to one of ordinary skill in the art.

As used herein, the term "sweetening agent" is intended to mean a compound used to impart sweetness to a preparation. Such compounds include, by way of example and without limitation, aspartame, dextrose, glycerin, mannitol, saccharin sodium, sorbitol and sucrose and other materials known to one of ordinary skill in the art.

As used herein, the term "antiadherent" is intended to mean an agent that prevents the sticking of solid dosage formulation ingredients to punches and dies in a tableting machine during production. Such compounds include, by way of example and without limitation, magnesium stearate, talc, calcium stearate, glyceryl behenate, PEG, hydrogenated vegetable oil, mineral oil, stearic acid and other materials known to one of ordinary skill in the art.

As used herein, the term " binder" is intended to mean a substance used to cause adhesion of powder particles in solid dosage formulations. Such compounds include, by way of example and without limitation, acacia, alginic acid, carboxymethylcellulose sodium, poly(vinylpyrrolidone), compressible sugar (e.g., NuTab), ethylcellulose, gelatin, liquid glucose, methylcellulose, povidone and pregelatinized starch and other materials known to one of ordinary skill in the art.

When needed, binders may also be included in the dosage forms. Exemplary binders include acacia, tragacanth, gelatin, starch, cellulose materials such as methyl cellulose and sodium carboxy methyl cellulose, alginic acids and salts thereof, polyethylene glycol, guar gum, polysaccharide, bentonites, sugars, invert sugars, poloxamers (PLURONIC™ F68, PLURONIC™ F127), collagen, albumin, gelatin, cellulosics in nonaqueous solvents, combinations thereof and others known to those of ordinary skill in the art. Other binders include, for example, polypropylene glycol, polyoxyethylene-polypropylene copolymer, polyethylene ester, polyethylene sorbitan ester, polyethylene oxide, combinations thereof and other materials known to one of ordinary skill in the art.

As used herein, the term " diluent" or "filler" is intended to mean an inert substance used as a filler to create the desired bulk, flow properties, and compression characteristics in the preparation of solid dosage forms. Such compounds include, by way of example and without limitation, dibasic calcium phosphate, kaolin, lactose, dextrose, magnesium carbonate, sucrose, mannitol, microcrystalline cellulose, powdered cellulose, precipitated calcium carbonate, sorbitol, and starch and other materials known to one of ordinary skill in the art.

As used herein, the term "direct compression excipient" is intended to mean a compound used in compressed solid dosage forms. Such compounds include, by way of example and without limitation, dibasic calcium phosphate (e.g., Ditab) and other materials known to one of ordinary skill in the art.

As used herein, a fragrance is a relatively volatile substance or combination of substances that produces a detectable aroma, odor or scent. Exemplary fragrances include those generally accepted as FD&C.

As used herein, the term " glidant" is intended to mean an agent used in solid dosage formulations to promote flowability of the solid mass. Such compounds include, by way of example and without limitation, colloidal silica, cornstarch, talc, calcium silicate, magnesium silicate, colloidal silicon, tribasic calcium phosphate, silicon hydrogel and other materials known to one of ordinary skill in the art.

As used herein, the term " lubricant" is intended to mean a substance used in solid dosage formulations to reduce friction during compression. Such compounds include, by way of example and without limitation, calcium stearate, magnesium stearate, PEG, talc, mineral oil, stearic acid, and zinc stearate and other materials known to one of ordinary skill in the art.

As used herein, the term "opaquant" is intended to mean a compound used to render a coating opaque. May be used alone or in combination with a colorant. Such compounds include, by way of example and without limitation, titanium dioxide, talc and other materials known to one of ordinary skill in the art.

As used herein, the term " polishing agent" is intended to mean a compound used to impart an attractive sheen to solid dosage forms. Such compounds include, by way of example and without limitation, carnauba wax, white wax and other materials known to one of ordinary skill in the art.

As used herein, the term "disintegrant" is intended to mean a compound used in solid dosage forms to promote the disruption of the solid mass into smaller particles which are more readily dispersed or dissolved. Exemplary disintegrants include, by way of example and without limitation, starches such as corn starch, potato starch, pre-gelatinized and modified starches thereof, sweeteners, clays, bentonite, microcrystalline cellulose(e.g., Avicel), carboxymethylcellulose calcium, croscarmellose sodium, alginic acid, sodium alginate, cellulose polyacrilin potassium (e.g., Amberlite), alginates, sodium starch glycolate, gums, agar, guar, locust bean, karaya, pectin, tragacanth, crospovidone and other materials known to one of ordinary skill in the art.

As used herein, the term "colorant" is intended to mean a compound used to impart color to solid (e.g., tablets) pharmaceutical preparations. Such compounds include, by way of example and without limitation, FD&C Red No. 3, FD&C Red No. 20, FD&C Yellow No. 6, FD&C Blue No. 2, D&C Green No. 5, D&C Orange No. 5, D&C Red No. 8, caramel, and ferric oxide, red, other F.D. & C. dyes and natural coloring agents such as grape skin extract, beet red powder, beta-carotene, annato, carmine, turmeric, paprika, and other materials known to one of ordinary skill in the art. The amount of coloring agent used will vary as desired.

As used herein, the term "flavorant" is intended to mean a compound used to impart a pleasant flavor and often odor to a pharmaceutical preparation. Exemplary flavoring agents or flavorants include synthetic flavor oils and flavoring aromatics and/or natural oils, extracts from plants, leaves, flowers, fruits and so forth and combinations thereof. These may also include cinnamon oil, oil of wintergreen, peppermint oils, clove oil, bay oil, anise oil, eucalyptus, thyme oil, cedar leave oil, oil of nutmeg, oil of sage, oil of bitter almonds and cassia oil. Other useful flavors include vanilla, citrus oil, including lemon, orange, grape, lime and grapefruit, and fruit essences, including apple, pear, peach, strawberry, raspberry, cherry, plum, pineapple, apricot and so forth. Flavors which have been found to be particularly useful include commercially available orange, grape, cherry and bubble gum flavors and mixtures thereof. The amount of flavoring may depend on a number of factors, including the organoleptic effect desired. Flavors will be present in any amount as desired by those of ordinary skill in the art. Particularly flavors are the grape and cherry flavors and citrus flavors such as orange.

As used herein, a preservative is an agent or combination of agents that inhibits, reduces or eliminates bacterial growth in a pharmaceutical dosage form. Exemplary preservatives includes Nipagin, Nipasol, Isopropyl alcohol and a combination thereof.

The present solid dosage form can also employ one or more commonly known surface active agents or cosolvents that improve wetting or disintegration of the core and/or layer of the solid dosage form.

Plasticizers can also be included in the tablets to modify the properties and characteristics of the polymers used in the coats or core of the tablets. As used herein, the term "plasticizer" includes all compounds capable of plasticizing or softening a polymer or binder used in invention. The plasticizer should be able to lower the melting temperature or glass transition temperature (softening point temperature) of the polymer or binder. Plasticizers, such as low molecular weight PEG, generally broaden the average molecular weight of a polymer in which they are included thereby lowering its glass transition temperature or softening point. Plasticizers also generally reduce the viscosity of a polymer. It is possible the plasticizer will impart some particularly advantageous physical properties to the osmotic device of the invention.

Plasticizers useful in the invention can include, by way of example and without limitation, low molecular weight polymers, oligomers, copolymers, oils, small organic molecules, low molecular weight polyols having aliphatic hydroxyls, ester-type plasticizers, glycol ethers, poly(propylene glycol), multi-block polymers, single block polymers, low molecular weight poly(ethylene glycol), citrate ester-type plasticizers, triacetin, propylene glycol and glycerin. Such plasticizers can also include ethylene glycol, 1,2-butylene glycol, 2,3-butylene glycol, styrene glycol, diethylene glycol, triethylene glycol, tetraethylene glycol and other polyethylene glycol) compounds, monopropylene glycol monoisopropyl ether, propylene glycol monoethyl ether, ethylene glycol monoethyl ether, diethylene glycol monoethyl ether, sorbitol lactate, ethyl lactate, butyl lactate, ethyl glycolate, dibutylsebacate, acetyltributylcitrate, triethyl citrate, acetyl triethyl citrate, tributyl citrate and allyl glycolate. All such plasticizers are commercially available from sources such as Aldrich or Sigma Chemical Co. It is also contemplated and within the scope of the invention, that a combination of plasticizers may be used in the present formulation. The PEG based plasticizers are available commercially or can be made by a variety of methods, such as disclosed in *Poly(ethylene glycol) Chemistry: Biotechnical* *and Biomedical Applications* (J.M. Harris, Ed.; Plenum Press, NY) the disclosure of which is hereby incorporated by reference.

The dosage form of the invention can also include oils, for example, fixed oils, such as peanut oil, sesame oil, cottonseed oil, corn oil and olive oil; fatty acids, such as oleic acid, stearic acid and isotearic acid; and fatty acid esters, such as ethyl oleate, isopropyl myristate, fatty acid glycerides and acetylated fatty acid glycerides. It can also be mixed with alcohols, such as ethanol, isopropanol, hexadecyl alcohol, glycerol and propylene glycol; with glycerol ketals, such as 2,2-dimethyl-1,3-dioxolane-4-methanol; with ethers, such as poly(ethyleneglycol) 450, with petroleum hydrocarbons, such as mineral oil and petrolatum; with water, or with mixtures thereof; with or without the addition of a pharmaceutically suitable surfactant, suspending agent or emulsifying agent.

Soaps and synthetic detergents may be employed as surfactants and as vehicles for pharmaceuical compositions. Suitable soaps include fatty acid alkali metal, ammonium, and triethanolamine salts. Suitable detergents include cationic detergents, for example, dimethyl dialkyl ammonium halides, alkyl pyridinium halides, and alkylamine acetates; anionic detergents, for example, alkyl, aryl and olefin sulfonates, alkyl, olefin, ether and monoglyceride sulfates, and sulfosuccinates; nonionic detergents, for example, fatty amine oxides, fatty acid alkanolamides, and poly(oxyethylene)-*block*-poly(oxypropylene) copolymers; and amphoteric detergents, for example, alkyl β-aminopropionates and 2-alkylimidazoline quaternary ammonium salts; and mixtures thereof.

Various other components, not otherwise listed above, can be added to the present formulation for optimization of a desired active agent release profile including, by way of example and without limitation, glycerylmonostearate, nylon, cellulose acetate butyrate, d, 1-poly(lactic acid), 1,6 - hexanediamine, diethylenetriamine, starches, derivatized starches, acetylated monoglycerides, gelatin coacervates, poly (styrene - maleic acid) copolymer, glycowax, castor wax, stearyl alcohol, glycerol palmitostearate, poly(ethylene), poly(vinyl acetate), poly(vinyl chloride), 1,3 - butylene-glycoldimethacrylate, ethyleneglycol-dimethacrylate and methacrylate hydrogels.

It should be understood, that compounds used in the art of pharmaceutical formulation generally serve a variety of functions or purposes. Thus, if a compound named herein is mentioned only once or is used to define more than one term herein, its purpose or function should not be construed as being limited solely to that named purpose(s) or function(s).

The amount of therapeutic compound incorporated in each osmotic device will be at least one or more unit dose and can be selected according to known principles of pharmacy. An effective amount of therapeutic compound is specifically contemplated. By the term "effective amount", it is understood that a therapeutically effective amount is contemplated. A therapeutically effective amount is the amount or quantity of COX-II inhibitor or muscle relaxant that is sufficient to elicit the required or desired therapeutic response, or in other words, the amount which is sufficient to elicit an appreciable biological response when administered to a patient.

The term "unit dose" is used herein to mean a dosage form containing a quantity of the therapeutic compound, said quantity being such that one or more predetermined units may be provided as a single therapeutic administration. Depending upon the specific combination and amounts of COX-II inhibitor and muscle relaxant included within the dosage form, an improved, additive or synergistic therapeutic effect will be observed. An improved therapeutic effect is one wherein the muscle relaxant enhances the therapeutic benefit (analgesic effect) provided by the COX-II inhibitor alone. An additive therapeutic effect is one wherein each of the muscle relaxant and the COX-II inhibitor possesses analgesic properties, and the combination of the two drugs provides an overall analgesic effect that approximates the sum of their individual analgesic effects. A synergistic therapeutic effect is one wherein each of the muscle relaxant and the COX-II inhibitor possesses analgesic properties, and the combination of the two drugs provides an overall analgesic effect that is greater the sum of their individual analgesic effects. In each embodiment of the invention, a particular combination of drugs will provide at least an improved therapeutic effect as compared to the individual drugs.

For nasal administration, the pharmaceutical composition may be included in a paste, cream, spray, powder, nebulizer, aerosol or ointment containing the appropriate solvents (such as water, aqueous, nonaqueous, polar, apolar, hydrophobic, hydrophilic and/or combinations thereof) and optionally other compounds (stabilizers, perfumes, antimicrobial agents, antioxidants, pH modifiers, surfactants and/or bioavailability modifiers). It is contemplated that bioavailability enhancers such as alcohols or other compounds that enhance the penetration of the therapeutic compound from the pharmaceutical formulation into the nasal mucosa may be needed to prepare suitable formulations for nasal administration.

For oral, buccal, and sublingual administration, the pharmaceutical composition may be in the form of a caplet, tablet, suspension, agglomerate, granulate or powder.

For rectal administration, the pharmaceutical composition can be included in a suppository, ointment, enema, tablet or cream for release of a therapeutic compound into the intestines, sigmoid flexure and/or rectum.

The solid dosage formulations of the invention can assume any shape or form known in the art of pharmaceutical sciences. The device of the invention can be a pill, sphere, tablet, bar, plate, paraboloid of revolution, ellipsoid of revolution or others known to those of ordinary skill in the art. The tablets can also include surface markings, cuttings, grooves, letters and/or numerals for the purposes of decoration, identification and/or other purposes.

The tablets of the invention can be prepared according to the methods disclosed herein or those well known in the art, more specifically according to the methods disclosed in the disclosure incorporated herein by reference. For example, according to one manufacturing technique, drug and excipients that comprise the core are mixed in solid, semisolid or gelatinous form, then moistened and sieved through a specified screen to obtain granules. The granules are then dried in a dryer and compressed, for example, by punching to form uncoated cores. The compressed and uncoated cores are then covered with a semipermeable membrane. Subsequently, the semipermeable membrane surrounding the core should be perforated with, for example, laser equipment. Finally, an external coat containing the drug is applied to the semipermeable membrane.

An external coat can be applied to a solid substrate as a compression coating, but it is generally applied as a sprayed coating. The sprayed coating is thinner and lighter than the compression coating, and a solid dosage form including the sprayed on external coating is, therefore, smaller than a similar solid dosage form having a compression coat. Moreover, the use of a sprayed-on drug-containing water soluble or erodible coating permits the loading of higher amounts of drug than the use of a compression-coated drug-containing water soluble coating. A smaller size solid dosage form generally results in increased patient compliance in taking the solid dosage form and is therefore advantageous.

The solid dosage form of the invention can be coated with a finish coat as is commonly done in the art to provide the desired shine, color, taste or other aesthetic characteristics. Materials suitable for preparing the finish coat are well known in the art and found in the disclosures of many of the references cited and incorporated by reference herein.

The following examples should not be considered exhaustive, but merely illustrative of only a few of the many embodiments contemplated by the present invention. The methods described herein can be followed to prepare solid dosage forms according to the invention.

### EXAMPLE 1

The following general method was used to prepare compressed tablets that provide an immediate release of a COX-II inhibitor and a muscle relaxant. Ferric oxide and 30% of the total lactose monohydrate were mixed and then sieved through a 200 mesh screen to form a blend. The blend was mixed with the remaining amount of lactose monohydrate, pridinol methanesulfonate, sodium lauryl sulfate, microcrystalline cellulose, croscarmellose sodium, rofecoxib and colloidal silicon dioxide to homogeneity to form a second blend. The second blend was mixed with 50% of the total magnesium stearate and the mixture tabletted to form 1 g cores. The cores were milled and mixed with the remaining magnesium stearate. The final mixture was tabletted to form 200 mg compressed tablets.

The above-detailed process was used to prepare tablets containing the following ingredients present in the approximate amounts indicated. FIGS. 1-2 depict the *in vitro* dissolution profiles for pridinol and rofecoxib as they are released from this tablet.

| INGREDIENT | APPROXIMATE AMOUNT (mg) |
|---|---|
| Rofecoxib | 25 |
| Pridinol methanesulfonate | 4 |
| Microcrystalline cellulose | 78.34 |
| Lactose monohydrate | 75 |
| Croscarmellose sodium | 8 |
| Ferric oxide | 0.66 |
| Magnesium stearate | 1 |
| Colloidal silicon dioxide | 4 |
| Sodium lauryl sulfate or ducosate sodium | 4 |

### EXAMPLE 2

The following general composition is used to prepare compressed tablets that provide an immediate release of a COX-II inhibitor and a sustained release of muscle relaxant. The following ingredients in the approximate amounts indicated were used to manufacture the tablets. The water is included during the manufacture but is not present in a significant amount in the finished tablet.

| INGREDIENT | AMOUNT (mg) | AMOUNT (mg) | AMOUNT (mg) |
|---|---|---|---|
| Rofecoxib | 10-15 | 20-35 | 35-60 |
| Pridinol | 0.1-5.0 | 0.1-5.0 | 5.0-10 |
| Hydroxypropylcellulose | 5-20 | 5-20 | 5-20 |
| Glyceryl monoestearate (Myvaplex 600 P) | 7-15 | 10-20 | 20-40 |
| Sodium croscarmellose | 10-30 | 10-30 | 10-30 |
| Lactose monohydrate | 70-300 | 70-300 | 70-300 |
| Purified water | 5.00 | 5.00 | 5.00 |
| Magnesium stearate | 1.5-6 | 1.5-6 | 1.5-6 |

The tablets are prepared as follows. Pridinol and Myvaplex 600 P are thoroughly mixed and melt extruded through a 12 USP mesh sieve. The extrudate is spheronized and sieved through a USP 12 mesh. The lactose monohydrate and rofecoxib are mixed and granulated with the hydroxypropylcellulose previously dissolved in purified water. The granules are dried and sieve through a USP 12 mesh screen. The dried granules are blended with the pridinol-containing beads, sodium croscarmellose and magnesium stearate. No. "0" sized capsules are then filled to final weight.

### EXAMPLE 3

The following general composition is used to prepare an osmotic device that provides an immediate release of a COX-II inhibitor and a controlled release of muscle relaxant. A scale batch was prepared by mixing 8.00 g of pridinol mesylate, 100.00 g of mannitol, 55.00 g of microcrystalline cellulose and 12.0 g of povidone. The mixture was wetted with a blend of 80.00 ml of alcohol (96°) and 100.0 g of PEG 400. The wet blend was granulated and dried at 40-50°C for 2 hours. The dried granulate was then screened and mixed with 2.00 g of colloidal silicon dioxide. This mixture was then mixed to homogeneity with 3.00 g of magnesium stearate. The final blend was tabletted using biconcave 9.00 mm diameter punches. The final core weight is about 190.0 mg with a hardness of about 8-14 kP.

A first composition (forming the semipermeable membrane) was prepared by mixing cellulose acetate (22.80 g) and PEG 400 (1.20 g) in a mixture of 490 ml of acetone and 200 ml of methyl alcohol. This polymer mixture was sprayed onto the tablet cores in a conventional pan coater to obtain film-coated tablets which membranes weighed about 24.0 mg. A 0.50 mm diameter hole was then drilled through one face of the tablet with a laser.

A second composition (forming an inert water soluble coat) was prepared by mixing copolyvidone (1.95 g), titanium dioxide (1.75 g), talc (6.25 g), and Aluminum Lake Ponceau Red (50.00 mg) in isopropyl alcohol. This polymer mixture was sprayed onto the semipermeable membrane coated tablets in a conventional pan coater to obtain film-coated tablets which membranes weight about 10 mg.

A third composition (forming a drug-containing coat) was prepared by mixing rofecoxib (25.00 g), microcrystalline cellulose (229.00 g), lactose monohydrate (166.3 g), corn starch (50.00 g) and povidone (20.00 g). This mixture was wetted with a mixture of alcohol (96°, 80.00 ml) and Polysorbate 20 (1.40 g). This wet mixture was then granulated and dried at 40-50°C for 3 hours. The dried granulate was screened and mixed with colloidal silicon dioxide (3.80 g). This mixture was mixed to homogeneity with magnesium stearate (4.50 g). This final mixture was then compressed about the inert coat using biconcave 14.00 mm diameter punches. The coat had a final weight of about 500.0 mg and a hardness of about 8-12 kP.

A final composition (for forming the finish coat) was prepared by mixing HPMC 2910 (9.10 g), PEG 6000 (2.56 g), and titanium dioxide (3.36 g) in a mixture of methylene chloride and alcohol (96°) (70:30 v/v). The final composition was sprayed onto the drug-containing coat in a conventional pan coater to obtain film-coated tablets which membranes weigh about 15 mg.

### EXAMPLE 4

The following general composition is used to prepare a suppository dosage form containing a COX-II inhibitor and a muscle relaxant. This dosage form is used for rectal or vaginal administration for the treatment of pain. The following ingredients are used in the approximate amounts indicated.

| INGREDIENT | Amount (mg) | Amount (mg) | Amount (mg) |
|---|---|---|---|
| Rofecoxib | 12.5 | 25.0 | 50.0 |
| Pridinol Methanesulfonate | 6.0 | 6.0 | 6.0 |
| Butylhydroxytoluene | 11.10 | 11.10 | 11.10 |
| Cremophor RH 40 | 222.0 | 222.0 | 222.0 |
| Macrogol 1500 (PhEur) | 1078.4 | 1075.9 | 1060.9 |
| Macrogol 4000 (PhEur) | 670.0 | 660.0 | 650.0 |

The suppository is prepared as follows. Butylhydroxytoluene is dissolved in warm Cremophor RH 40. Rofecoxib and pridinol methansulfonate are mixed and added with continuous stirring. The mixture is then blended with molten Lutrol E (grades 1500 and 4000). The molten mixture is then filled into suppository molds to obtain suppositories weighing about 2 grams.

### EXAMPLE 5

The following general composition is used to prepare a cream dosage form containing a COX-II inhibitor and a muscle relaxant. This dosage form is used for topical administration for the treatment of muscle or joint pain. The following ingredients are used in the approximate amounts indicated.

| INGREDIENT | AMOUNT (mg) |
|---|---|
| Rofecoxib | 5.00 |
| Pridinol methanesulfonate | 2.00 |
| Glyceryl monostearate | 760.0 |
| Poloxamer (Pluronic F68) | 9.25 |
| Refreshing Fragrance | 0.06 |
| Methylparaben | 0.04 |
| Propylparaben | 0.02 |
| Mineral Oil | 180.63 |
| Purified Water q.s. | 0.05 |

The cream is prepared as follows. The Pluronic F 68 is dissolved in purified water and heated to 50°C. The rofecoxib is added and the mixture is stirred for 30 minutes.

The glyceryl monostearate is heated until completely molten. Mineral oil is added to the glyceryl monostearate while keeping the temperature at about 65°C. The mixture is cooled to about 50°C and pridinol methanesulfonate is added.

Sodium methylparaben and sodium propylparaben are dissolved in water heated to 60°C. This mixture is added to the rofecoxib-containing mixture with stirring. This mixture is then added to the pridinol-containing mixture. After the temperature has dropped to about 25°, a refreshing fragrance is added.

### EXAMPLE 6

The following general composition is used to prepare a gel dosage form containing a COX-II inhibitor and a muscle relaxant. This dosage form is used for topical administration for the treatment of muscle or joint pain. The following ingredients are used in the approximate amounts indicated.

| INGREDIENT | AMOUNT (g) |
|---|---|
| Rofecoxib | 0.0050 |
| Pridinol methanesulfonate | 0.0020 |
| Propylene glycol | 0.050 |
| Isopropyl Alcohol | 0.150 |
| Carbomer (Carbopol Ultrez 10) | 0.008 |
| Triethanolamime | 0.0087 |
| Edetate Disodium | 0.001 |
| Purified Water | 0.777 |
| refreshing Fragrance | 0.0006 |

The above gel is manufactured as follows. The rofecoxib and pridinol methanesulfonate are dispersed in a mixture of propylene glycol and 97% Isopropyl alcohol. A refreshing fragrance is dissolved in the remaining isopropyl alcohol. The edetate disodium is dissolved in purified water at 45°C. Carbomer is added to the water while stirring slowly and letting the solution cool to 20-25°C. The rofecoxib mixture and carbomer mixture are blended with stirring over a period of about 30 minutes at 800-1000 rpm. Triethanolamine is added while stirring to obtain a homogenous mixture.

### EXAMPLE 7

The following general composition is used to prepare an ointment dosage form containing a COX-II inhibitor and a muscle relaxant. This dosage form is used for topical administration for the treatment of muscle or joint pain. The following ingredients are used in the approximate amounts indicated.

| INGREDIENT | AMOUNT (g) |
|---|---|
| Rofecoxib | 0.005 |
| Pridinol methanesulfonate | 0.002 |
| Methylparaben | 0.0003 |
| Propylparaben | 0.0001 |
| Sodium lauryl Sulfate | 0.010 |
| Propylene Glycol | 0.120 |
| Stearyl Alcohol | 0.250 |
| White Petrolatum | 0.250 |
| Purified Water | 0.970 |
| Refreshing Fragrance | 0.06 mg |

The ointment is made as follows. Stearyl alcohol and the white petrolatum are melted on a steam bath and warmed to about 75°C. The rofecoxib and pridinol methanesulfonate are dispersed in propylene glycol along with methylparaben and propylparaben and warmed to 50°C. The sodium lauryl sulfate is dissolved in purified water and warmed to 75°C. The lauryl sulfate-containing solution is added to the molten petrolatum mixture while heating. Finally, the rofecoxib-pridinol dispersion is added to the mixture to form an emulsion that is cooled to 45°C before adding the refreshing fragrance.

### EXAMPLE 8

The following general composition is used to prepare a suspension dosage form containing a COX-II inhibitor and a muscle relaxant. This dosage form is used for oral administration for the treatment of pain. The following ingredients are used in the approximate amounts indicated.

| Ingredient | Amount (mg) |
|---|---|
| Rofecoxib | 25 |
| Pridinol mesylate | 4 |
| Poloxamer (Pluronic F68) | 9.25 |
| Methylparaben | 0.03 |
| Propylparaben | 0.01 |
| Citric acid (monohydrate) | 317 |
| Strawberry flavorant | 0.02 ml |
| Sodium citrate (dihydrate) | 475 |
| Xanthan gum | 6.5 |
| Purified water q.s. | 2 ml |

The suspension is made as follows. The Pluronic F68 is dissolved in the purified water which is heated to 50° C. The rofecoxib is then added and stirred for about 30 min. The citric acid, sodium citrate and xanthan gum are dissolved in the purified water and the pH adjusted as desired. The pridinol is then added to the citrate-containing solution. In a quarter portion of the water heated to 60° C, the sodium methylparaben and sodium propylparaben are dissolved. The rofecoxib-containing mixture is added and then the pridinol-containing mixture is added. Once the temperature has reached about 25° C, the strawberry flavorant is added.

### EXAMPLE 9

The following general composition is used to prepare an injectable dosage form containing a COX-II inhibitor and a muscle relaxant. This dosage form is used for parenteral administration for the treatment of pain. The following ingredients are used in the approximate amounts indicated.

| INGREDIENT | AMOUNT (mg) |
|---|---|
| Rofecoxib | 25 |
| Pridinol mesylate | 2.2 |
| Poloxamer (Pluronic F68) | 9.25 |
| Propylene glycol | 1.8 |
| Glacial acetic acid | 0.03 ml |
| Sodium hydroxide | 0.02 ml |
| Water q.s. | 5.0 ml |

The injectable is made as follows. The Pluronic F68 is dissolved in the water which is heated to 50° C. The rofecoxib is added and stirred for about 30 min. The propylene glycol is added with stirring until complete dissolution. The pridinol mesylate is dissolved in water and its pH adjusted to about 7 with glacial acetic acid and sodium hydroxide. The two solutions are mixed and the pH adjusted as needed. The mixed solution is filtered through a 0.22 micron filter and into a sterile container. The solution is then filled into sterile 5 ml ampoules.

### EXAMPLE 10

The following general procedure was used to evaluate the performance of a pharmaceutical composition according to the invention. A single-dose study was conducted using a tablet made according to Example 1. The tablet contained rofecoxib (25 mg) and pridinol (4 mg) both in immediate release forms. Single tablets were administered to 12 healthy patients in a single-dose open-label, randomized pharmacokinetic study by performing a comparison of its concentration-time curve with that of a commercial product (25 mg rofecoxib) as control. The mean plasma concentration obtained, the standard deviation, and minimum and maximum observed plasma concentrations at specific time points were determined. The formulation of the invention and the commercial product provided similar plasma concentration profiles and total areas under the curve for rofecoxib. FIGS. 3-4 depict the mean and minimum-maximum plasma profiles for rofecoxib in the formulation of the invention.

The following table shows the pharmacokinetic parameters of rofecoxib in the example 1 compared to the commercial product, and shows that the pridinol, in the formulation of the invention, did not change either the extent of absorption or the pharmacokinetic profile of rofecoxib, indicating similar efficacy between both products. Moreover, none of the subjects presented any adverse events, indicating the safety of the administration of the product, depicted in the example 1.

| Parameter | Example 1 Media + sd (n+12) | Commercial product Media + sd (n-12) |
|---|---|---|
| Cmax (ng/ml) | 155.7 +/- 28.3 | 137.1 +/- 20.6 |
| AUCinf(ng.h/ml | 3736.5 +/- 951.0 | 3782.3 +/- 1033.2 |
| AUC0-t (ng.h/ml) | 2424.4 +/- 802.4 | 3362.5 +/- 763.3 |
| Tmax (h) | 5.8 +/- 2.9 | 8.7 +/- 5.0 |
| Ke (h-1) | 0.05953 +/- 0.0133 | 0.05735 +/- 0.0150 |
| t1/2 (h) | 12.2 +/- 2.9 | 12.9 +/- 3.7 |

### EXAMPLE 11

The following general composition is used to prepare two different solid dosage forms containing a COX-II inhibitor and a muscle relaxant. This dosage form is used for oral administration for the treatment of pain. The tablets below provide an immediate or rapid release of the rofecoxib and a sustained release of the pridinol. The following ingredients are used in the approximate amounts indicated.

| Ingredient | Amount (mg) | Amount (mg) |
|---|---|---|
| Rofecoxib | 25 | 50 |
| Pridinol | 4 | 8 |
| HPC type LH 11 or LH 21 (low degree of substitution | 9.25 | 9.25 |
| Glyceryl monostearate (Myvaplex 600 P) | 15 | 30 |
| Sodium Croscarmellose | 18 | 18 |
| Lactose monohydrate | 125.75 | 81.75 |
| Purified water | 5 ml | 5 ml |
| Magnesium stearate | 3 | 3 |

### EXAMPLE 12

The following general composition is used to prepare immediate release capsules that provide an immediate release of a COX-II inhibitor and a muscle relaxant. This dosage form is used for oral administration for the treatment of pain. The following ingredients are used in the approximate amounts indicated.

| INGREDIENT | AMOUNT (mg) | AMOUNT (mg) |
|---|---|---|
| Rofecoxib | 25.00 | 50.00 |
| Pridinol | 4.00 | 8.00 |
| Hydroxypropylcellulose (*) | 9.25 | 9.25 |
| Microcrystalline Cellulose PH 301 | 136.00 | 150.75 |
| Lactose Monohydrate | 125.75 | 81.75 |
| Purified Water | 5.00 | 5.00 |

| | | |
|---|---|---|
| * indicates HPC having a low degree of substitution; generally type LH 11 or LH 21. | | |

The capsule is made as follows. The lactose monohydrate, pridinol, and rofecoxib are weighed, mixed and granulated with the hydroxypropylcellulose that has been previously dissolved in purified water. The wet granules are dried, passed through a USP 12 mesh sieve, and blended with microcrystalline cellulose PH 301. No. "0" size capsules are then filled to the desired final capsule weight with the granules and MCC.

### EXAMPLE 13

The following general composition is used to prepare an osmotic device that provides an immediate release of a COX-II inhibitor and a controlled release of muscle relaxant. A scale batch was prepared by mixing 8.00 g of pridinol mesylate, 100.00 g of mannitol, 55.00 g of microcrystalline cellulose and 12.0 g of povidone. The mixture was wetted with a blend of 80.00 ml of alcohol (96°) and 100.0 g of PEG 400. The wet blend was granulated and dried at 40-50°C for 2 hours. The dried granulate was then screened and mixed with 2.00 g of colloidal silicon dioxide. This mixture was then mixed to homogeneity with 3.00 g of magnesium stearate. The final blend was tabletted using biconcave 9.00 mm diameter punches. The final core weight is about 190.0 mg with a hardness of about 8-14 kP.

A first composition (forming the semipermeable membrane) was prepared by mixing cellulose acetate (22.80 g) and PEG 400 (1.20 g) in a mixture of 490 ml of acetone and 200 ml of methyl alcohol. This polymer mixture was sprayed onto the tablet cores in a conventional pan coater to obtain film-coated tablets which membranes weighed about 24.0 mg. A 0.50 mm diameter hole was then drilled through one face of the tablet with a laser.

A second composition (forming an inert water soluble coat) was prepared by mixing copolyvidone (1.95 g), titanium dioxide (1.75 g), talc (6.25 g), and Aluminum Lake Ponceau Red (50.00 mg) in isopropyl alcohol. This polymer mixture was sprayed onto the semipermeable membrane coated tablets in a conventional pan coater to obtain film-coated tablets which membranes weight about 10 mg.

A third composition (forming a drug-containing coat) was prepared by mixing rofecoxib (50.00 g), microcrystalline cellulose (250.00 g), lactose monohydrate (177.6 g), corn starch (57.00 g) and povidone (25.00 g). This mixture was wetted with a mixture of alcohol (96°, 100.00 ml) and Polysorbate 20 (1.60 g). This wet mixture was then granulated and dried at 40-50°C for 3 hours. The dried granulate was screened and mixed with colloidal silicon dioxide (4.10 g). This mixture was mixed to homogeneity with magnesium stearate (4.70 g). This final mixture was then compressed about the inert coat using biconcave 14.50 mm diameter punches. The coat had a final weight of about 570.0 mg and a hardness of about 7-12 kP.

A final composition (for forming the finish coat) was prepared by mixing HPMC 2910 (12.10 g), PEG 6000 (3.41 g), and titanium dioxide (4.48 g) in a mixture of methylene chloride and alcohol (96°) (70:30 v/v). The final composition was sprayed onto the drug-containing coat in a conventional pan coater to obtain film-coated tablets which membranes weigh about 20 mg.

The above is a detailed description of particular embodiments of the invention. It is recognized that departures from the disclosed embodiments may be made within the scope of the invention and that obvious modifications will occur to a person skilled in the art. Those of skill in the art should, in light of the present disclosure, appreciate that many changes can be made in the specific embodiments which are disclosed herein and still obtain a like or similar result without departing from the spirit and scope of the invention. All of the embodiments disclosed and claimed herein can be made and executed without undue experimentation in light of the present disclosure.

## Claims

1. A pharmaceutical composition comprising:
a) a COX-II inhibitor;
b) a muscle relaxant; and
c) at least one pharmaceutical excipient.

2. The pharmaceutical composition of claim 1, wherein the COX-II inhibitor binds COX-II receptors selectively over COX-I receptors.

3. The pharmaceutical composition of claim 1, wherein the COX-II inhibitor binds COX-II receptors specifically.

4. The pharmaceutical composition of claim 1, wherein the weight ratio of COX-II inhibitor to muscle relaxant varies from (12.5:2.2) to (50:8).

5. The pharmaceutical composition of claim 1, wherein the COX-II inhibitor is selected from the group consisting of central muscle relaxants and neuromuscular blocking agents.

6. The pharmaceutical composition of claim 1, wherein the at least one pharmaceutical excipient is independently selected from the group consisting of an acidifying agent, adsorbents, alkalizing agent, antioxidants, buffering agent, colorant, flavorant, sweetening agent, tablet antiadherent, tablet binder, tablet and capsule diluent, tablet direct compression excipient, tablet disintegrant, tablet glidant, tablet lubricant, tablet or capsule opaquant, plasticizer, surface active agent, solvent, oil, soap, detergent, and tablet polishing agent.

7. The pharmaceutical composition of claim 1, wherein the muscle relaxant is selected from the group consisting of alcuronium, alosetron, aminophylline, baclofen, carisoprodol, chlorphenesin, chlorphenesin carbamate, chlorzoxazone, chlormezanone, cyclobenzaprine, dantrolene, decamethonium, diazepam, dyphylline, eperisione, ethaverine, gallamine triethiodide, hexafluorenium, mephenesin, metaxalone, methocarbamol, metocurine iodide, orphenadrine, pancuronium, papaverine, pipecuronium, pridinol, succinylcholine, theophylline, tizanidine, tolperisone, tubocurarine, vecuronium, idrocilamide, ligustilide, cnidilide, and senkyunolide.

8. The pharmaceutical composition of claim 1 or 7, wherein the COX-II inhibitor is selected from the group consisting of rofecoxib, celecoxib, flosulide, NS-398, DUP-697, meloxicam, 6-methoxy-2-naphthylacetic acid, nabumetone, etodolac, nimesulide, SC-5766, SC-58215, T-614, and combinations thereof.

9. A method of treating pain in a mammal comprising the step of administering a pharmaceutical composition according to any one of claims 1-7, wherein the pharmaceutical composition provides therapeutically effective levels of each drug when administered to a mammal.

10. A pharmaceutical dosage form comprising:
a) a therapeutically effective amount of a COX-II inhibitor;
b) a therapeutically effective amount of a muscle relaxant; and
c) at least one pharmaceutical excipient.

11. The pharmaceutical dosage form of claim 10, wherein the dosage form is selected from the group consisting of a gel, cream, ointment, pill, tablet, capsule, liquid, suspension, osmotic device, bead, granule, spheroid, particulate, paste, reconstitutable solid, powder, and injectable liquid.

12. The pharmaceutical dosage form of claim 10, wherein the dosage form independently provides a controlled, delayed, sustained, immediate, timed, slow or rapid release of each of the COX-II inhibitor and the muscle relaxant when exposed to an aqueous environment.

13. The pharmaceutical dosage form of claim 10, wherein the dosage form provides therapeutically effective plasma levels of the COX-II inhibitor for a period up to at least about 12 hours after administration to a subject.

14. The pharmaceutical dosage form of claim 10, wherein after administration to a subject the dosage form provides therapeutically effective plasma levels of the muscle relaxant for a period of administration sufficient to enhance the therapeutic benefit provided by the COX-II inhibitor.

15. The pharmaceutical dosage form of claim 10, wherein the pharmaceutical dosage form is adapted for oral, buccal, ocular, otic, gastrointestinal, dermal, rectal, vaginal, cervical, intrauterine, epidermal, transdermal, implant, mucosal, parenteral, sublingual, nasal, or pulmonary delivery.

16. The pharmaceutical dosage form of claim 10, wherein the muscle relaxant is selected from the group consisting of alcuronium, alosetron, aminophylline, baclofen, carisoprodol, chlorphenesin, chlorphenesin carbamate, chlorzoxazone, chlormezanone, cyclobenzaprine, dantrolene, decamethonium, diazepam, dyphylline, eperisione, ethaverine, gallamine triethiodide, hexafluorenium, mephenesin, metaxalone, methocarbamol, metocurine iodide, orphenadrine, pancuronium, papaverine, pipecuronium, pridinol, succinylcholine, theophylline, tizanidine, tolperisone, tubocurarine, vecuronium, idrocilamide, ligustilide, cnidilide, and senkyunolide.

17. The pharmaceutical dosage form of claim 10 or 16, wherein the COX-II inhibitor is selected from the group consisting of rofecoxib, celecoxib, flosulide, NS-398, DUP-697, meloxicam, 6-methoxy-2-naphthylacetic acid, nabumetone, etodolac, nimesulide, SC-5766, SC-58215, T-614, and combinations thereof.

18. The pharmaceutical dosage form of claim 10, wherein each drug is released rapidly and the dosage form provides therapeutically effective levels of each drug for a period of at least 12 hours after administration to a subject.

19. The pharmaceutical dosage form of claim 18, wherein the period is about 12 to 60 hours.

20. The pharmaceutical dosage form of claim 19, wherein the period is about 12 to 30 hours.

21. The pharmaceutical dosage form of claim 19, wherein the period is about 18 to 48 hours.

22. The pharmaceutical dosage form of claim 10, wherein after administration to a subject the plasma level of the COX-II inhibitor or muscle relaxant is dependent upon the plasma level of the muscle relaxant or COX-II inhibitor, respectively.

23. The pharmaceutical dosage form of claim 10, wherein after administration to a subject the plasma level of the COX-II inhibitor or muscle relaxant is independent of the plasma level of the muscle relaxant or COX-II inhibitor, respectively.

24. The pharmaceutical dosage form of claim 10, wherein the dosage form provides therapeutic plasma levels for the muscle relaxant in an amount sufficient to provide a therapeutic benefit to a subject to whom it is administered.

25. The pharmaceutical dosage form of claim 10, wherein after administration to a subject the dosage form provides therapeutic plasma levels for the COX-II inhibitor in the range of about 90 ng to about 300 ng per ml of plasma in the subject.

26. The pharmaceutical dosage form of claim 10, wherein the COX-II inhibitor and muscle relaxant are released sequentially after exposure to an aqueous environment.

27. The pharmaceutical dosage form of claim 10, wherein the COX-II inhibitor and muscle relaxant are released concurrently after exposure to an aqueous environment.

28. The pharmaceutical dosage form of claim 10, wherein the COX-II inhibitor and muscle relaxant are released in spaced apart periods of time after exposure to an aqueous environment.

29. The pharmaceutical dosage form of claim 10, wherein each drug is independently released according to a rapid, immediate, controlled, sustained, slow, timed, targeted, pseudo-first order, first order, pseudo-zero order, zero-order, and/or delayed release profile after exposure to an aqueous environment.

30. The pharmaceutical dosage form of claim 10, wherein the dosage form provides a controlled release of the COX-II inhibitor and a controlled release of the muscle relaxant after exposure to an aqueous environment.

31. The pharmaceutical dosage form of claim 10, wherein the dosage form provides a controlled release of the COX-II inhibitor and a rapid release of the muscle relaxant after exposure to an aqueous environment.

32. The pharmaceutical dosage form of claim 10, wherein the dosage form provides a controlled release of the muscle relaxant and a rapid release of the COX-II inhibitor after exposure to an aqueous environment.

33. The pharmaceutical dosage form of claim 10, wherein the dosage form provides a rapid release of the COX-II inhibitor and of the muscle relaxant after exposure to an aqueous environment.

34. The pharmaceutical dosage form of claim 10, wherein the dosage form provides a rapid release of the muscle relaxant and a delayed but rapid release of the COX-II inhibitor after exposure to an aqueous environment.

35. The pharmaceutical dosage form of claim 10, wherein the dosage form provides a rapid release of the muscle relaxant and a timed but controlled release of the COX-II inhibitor after exposure to an aqueous environment.

36. The pharmaceutical dosage form of claim 10, wherein the dosage form provides a rapid release of the COX-II inhibitor and a delayed but rapid release of the muscle relaxant after exposure to an aqueous environment.

37. The pharmaceutical dosage form of claim 10, wherein the dosage form provides a rapid release of the COX-II inhibitor and a timed but controlled release of the muscle relaxant after exposure to an aqueous environment.

38. The pharmaceutical dosage form of claim 10, wherein the weight ratio of COX-II inhibitor to muscle relaxant varies from 12.5:2.2 to 50:8.

39. A method of treating pain in a mammal comprising the step of administering a dosage form according to any one of claims 10-16, 18-37 or 38, wherein the dosage form provides therapeutically effective levels of each drug when administered to a mammal.

40. A pharmaceutical composition comprising:
a) a COX-II inhibitor selected from the group consisting of rofecoxib, celecoxib, flosulide, NS-398, DUP-697, meloxicam, 6-methoxy-2-naphthylacetic acid, nabumetone, etodolac, nimesulide, SC-5766, SC-58215, T-614, and combinations thereof;
b) a muscle relaxant selected from the group consisting of alcuronium, alosetron, aminophylline, baclofen, carisoprodol, chlorphenesin, chlorphenesin carbamate, chlorzoxazone, chlormezanone, cyclobenzaprine, dantrolene, decamethonium, diazepam, dyphylline, eperisione, ethaverine, gallamine triethiodide, hexafluorenium, mephenesin, metaxalone, methocarbamol, metocurine iodide, orphenadrine, pancuronium, papaverine, pipecuronium, pridinol, succinylcholine, theophylline, tizanidine, tolperisone, tubocurarine, vecuronium, idrocilamide, ligustilide, cnidilide, and senkyunolide and combinations thereof; and
c) at least one pharmaceutical excipient.

41. The composition of claim 40, wherein the at least one pharmaceutical excipient is independently selected from the group consisting of an acidifying agent, adsorbent, alkalizing agent, antioxidant, buffering agent, colorant, flavorant, sweetening agent, tablet antiadherent, tablet binder, tablet and capsule diluent, tablet direct compression excipient, tablet disintegrant, tablet glidant, tablet lubricant, tablet or capsule opaquant, plasticizer, surface active agent, solvent, oil, soap, detergent, and tablet polishing agent.

42. The composition of claim 41, the weight ratio of COX-II inhibitor to muscle relaxant varies from (12.5:2.2) to (50:8).

43. The composition of claim 40, wherein the COX-II inhibitor and muscle relaxant are independently provided in each occurrence in controlled, sustained, immediate, timed, slow or rapid release form.

44. The composition of claim 43, wherein at least one of the COX-II inhibitor and muscle relaxant are independently further provided in each occurrence in delayed or targeted release form.

45. The composition of claim 40, wherein at least one of the COX-II inhibitor and muscle relaxant are independently provided in each occurrence in pseudo-first order, first order, pseudo-zero order, or zero order release form.

46. A pharmaceutical dosage form comprising the pharmaceutical composition of claim 40.

47. A pharmaceutical dosage form comprising the pharmaceutical composition of claim 41.

48. A pharmaceutical dosage form comprising the pharmaceutical composition of claim 42.

49. A pharmaceutical composition comprising:
a) a COX-II inhibitor selected from the group consisting of rofecoxib and celecoxib;
b) pridinol; and
c) at least one pharmaceutical excipient.

50. The pharmaceutical composition of claim 49, wherein the at least one pharmaceutical excipient is independently selected from the group consisting of an acidifying agent, adsorbent, alkalizing agent, antioxidant, buffering agent, colorant, flavorant, sweetening agent, tablet antiadherent, tablet binder, tablet and capsule diluent, tablet direct compression excipient, tablet disintegrant, tablet glidant, tablet lubricant, tablet or capsule opaquant, plasticizer, surface active agent, solvent, oil, soap, detergent, and tablet polishing agent.

51. The composition of claim 49, the weight ratio of COX-II inhibitor to pridinol varies from (12.5:2.2) to (50:8).

52. The composition of claim 49, wherein the COX-II inhibitor and pridinol are independently provided in each occurrence in controlled, sustained, immediate, timed, slow or rapid release form.

53. The composition of claim 52, wherein at least one of the COX-II inhibitor and pridinol are independently further provided in each occurrence in delayed or targeted release form.

54. The composition of claim 49, wherein at least one of the COX-II inhibitor and pridinol are independently provided in each occurrence in pseudo-first order, first order, pseudo-zero order, or zero order release form.

55. A method of treating pain in a mammal comprising the step of administering a dosage form according to any one of claims 40-54, wherein the dosage form provides therapeutically effective levels of each drug when administered to a mammal.

56. A method of treating pain in a mammal comprising the step of administering a pharmaceutical composition according to claim 8, wherein the pharmaceutical composition provides therapeutically effective levels of each drug when administered to a mammal.

57. A method of treating pain in a mammal comprising the step of administering a dosage form according to claim 17, wherein the dosage form provides therapeutically effective levels of each drug when administered to a mammal.
